# EUROPEAN PATENT APPLICATION

(11) **EP 3 916 093 A1**
(43) Date of publication of application: **01.12.2021**
(21) Application number: 20181468.8
(22) Date of filing: 22.06.2020
(51) Int. Cl.: C12N 15/113, A61K 31/7105

(54) **RNA MOLECULES FOR THE TREATMENT OF CANCER**

(30) Priority: 29.05.2020 EP 20177607
(71) Applicant: ETH Zurich, 8092 Zürich (CH)
(72) Inventor: Paro, Renato, 4102 Binningen (CH); Birbaumer, Tosca, 4103 Bottmingen (CH); Jiang, Yanrui, 4052 Basel (CH); Schlumpf, Tommy Beat, 4553 Subingen (CH); Seimiya, Makiko, 4123 Allschwil (CH)
(74) Representative: Stolmár & Partner Patentanwälte PartG mbB

(57) **Abstract**

The invention is directed to a RNA molecule having a sequence having at least 80% sequence identity to SEQ ID NO: 2. Likewise, the invention is directed to a RNA molecule obtainable by transcription of a DNA sequence having at least 80% sequence identity to SEQ ID NO: 1 for use as a medicament and to pharmaceutical compositions comprising such a RNA molecule.

SEQUENCE LISTING

<110> ETH Zurich, ETH transfer, HG E 43-49

<120> RNA molecules for the treatment of cancer

<130> 38407-ETH-P-EP

<150> EP 20177607.7
<151> 2020-05-29

<160> 2

<170> PatentIn version 3.5

<210> 1
<211> 6157
<212> DNA
<213> Drosophila melanogaster

<220>
<221> misc_feature
<223> Drosophila melanogaster BDGP6.28, Chromosome 2L:
18,455,718-18,461,874

<400> 1
tttgcaaacg tatcgttgca cacggcggtt cgcgaatcgc gcttcctctt cccgctgcag 60
ctgtgtgcgt gttgtggtgt actcgtattg gcagcgcgta tgtgtgtgcg taactacact 120
gcaaattgtg acggattctt gtgttcctca gggggaaata acttgcgttt gacaggtgcg 180
atctagtgtg ccgtctcaaa aatgaaaaaa aacgatgtct ggttgcattc tctatttgcg 240
ccactggtaa gcgagaaaat tgattaaaat gcacattaca aagggggaaa tatattttaa 300
aaaaaatatg tagcttaaat cttaagtatt cccgtgtgtg tgtgtgtgtg tgtgtgtgac 360
agagagaacg agaaggagag agagagagaa cgagagagtg cagccagaaa ttgcaataca 420
cacaaacaaa ttgccacgca aaaagcggcc ggcttgccct cccccttccc tccgccattg 480
ctgccccccc cttcgtccat ccctttgtat gtgtgcattg gatgcacttg gtgcgattca 540
tttgctttta ttaccatttt tgcccgtctc tccactcttt tttttcttat tttgtggggc 600
cttctgcgcg atggcaaccc tggcagcaaa atgcgttggt gaataagttc tctacccttt 660
cgtcagtgtg ggacgcgact ttgaaatgct tggagctggc ggcaatgcag cgctatatgc 720
aataaaaacg tttataaata aaaaaataat atttactgaa atcgaagaac ttcccaatcg 780
gctaaacttt cgcaacgctt tgaaacggcg atcggacaaa agaaaaaaat taagaaaatg 840
tgcgtgaaat gtaaccaacg atttacaaca atagtaacac ttataataac aaattaataa 900
aacaatcaat aaatatatga caaacttgtt cgttttgtga gttctccata cctttctaat 960
ggatgtaata ggtctgggaa gtcctttaaa aaatgtataa aagtctatat ttgaaatgct 1020
gaactaacta tttagtagtg catttaaatc gatgcatttc agacaaaact tgtacaacta 1080
catattccag tgtttttacc tacacttaaa ttattaaatt gtaaggaatc gtgtttttgg 1140
gcacgttttt agtcgatttc agggctgagc aatttttaaa acctttttct atttaggata 1200

taaaatcaaa agagtttccc tctacaattt tcatgccgaa aaccaattga aggcaattaa 1260
aacgcatttt ctgtgcaatt gatgatgacc tacgacaaag gaaatgaagt catacccata 1320
cacacacgac tctcacgcac acatccacaa agctagaggc aaagtgcagg ccaaaagcga 1380
atgaagaaca aaaacaacaa gcaggggccc tacgaaagcc aacaacaaca aagtgcaaat 1440
tgcactgcgc atgcacatgc acaaacacac acacacacag gcaaccgcag cacacacaga 1500
tactccaact gaggcgaatt gcagtggtgt tcgggcgctt ttgttcgcct gcgaaatgaa 1560
aaaacagctc gaaaaagtac ttaaaaatat atttaaattt taaaaattac tcgaaaggct 1620
attccaaaat attcacaaaa ttggggaact ttaataaatt tcaatttttt ccgcctgcat 1680
tcctcctctt tctggcttcc tttatttata atattaaaat gcgcgtcatg ccaacgacag 1740
ttgaatgcac tcggcctttg ccttcaatgg cttccccctt gactgataat gataataaac 1800
agattattcg ctttttccaa tacgtttttg tattaaaagc aagctctcca gccgctagct 1860
tcactttgat gccacaaagc atgcttctta agcagttttc gatatttgag agtaaaaatc 1920
aactcttttt ttcaatattt ctagctgttc gtttcaaagc tgtttggatg tcattgaatt 1980
tcaatactta ctgaatacgc gttgatattc tattgtcttg acaatatcta catttatgaa 2040
tccatctcca aatatgtaat acgttaaaca gtatattctt atcctgacag agttgcatag 2100
tccacatttt atacgcttac tccgatatta tttatttagt tctgactcat agtccgacgg 2160
cgtttccttt cgaccttgag tgaagtattt aaagtagaat gggcagtgca attattaaat 2220
tctctgcgaa ccgcagagaa ttgcacttgg tggttgtcgt tctctgggcc gctggcttga 2280
gttgtgtatc tacaactaga attactcatc atcagcattt ccatagaaat cagaactcaa 2340
aacaataaac caaagtaagg aaggccgttt aattattaaa tagaaggctt ttatacaaca 2400
tttacttact taatctggtt aaaaactgca gtgaaaacta atttttaaaa aacaaaactt 2460
tttataacta taattgttaa aagagatttt aaaatgaaaa ctacataaca gaaaaagcga 2520
ttaaacttta tatataaatg atatctaaca aaggacctaa aaggaccata aacaggaatg 2580
ctaacaaaca taaaatcaaa gtaaatgtga atttatagaa tcctttcagg acttaaaaac 2640
tttgcaagca aaacggcaaa aatatacaag aaatgacagt acttttccta cttccttctt 2700
tcgtaccatt tgaactccat tttttttgtt gggcgagtgc aaaagggatg ggtagaggtg 2760
ccaaaggaca atggttcaag tgcaaagagg atgccaagcc gcatgcaaca atgccaacaa 2820
caaggaccga acgaacgccg aaggacatgc cgtgcaattt ccttttctgt ggtgagttgc 2880
agctattgcg tatacgccgt gtggtgtaca gagcgaaaga gaggccacta tgccgtctgg 2940
atagacagag agcgacgcta tttatagcag ggagctccaa aaccgagctc agccttgatt 3000
ccgcccttga actaacattg ctggggaact attcctgtct ctccagtcct gaccctttcc 3060
gtcccaaccc gtccattccc gatttcgtac ccttcgaatc gcccagcagt cgtcacagct 3120
ggaaaccagt tgggggagca catatgagga ggatgtgcca ggggaatact ggaggtacag 3180
gaggtacttg gggcatcgag aaccacagtg catatgctgc cagtcaagcg gtaattttga 3240
gtttgcagtt cgctctttcg cactgtccca tgcctcatgc cccatgctcc atgccccatg 3300
ctccatgtcc catgtccttg ccccaccaaa ctgaactcag caggctttga cattgatttt 3360
gtgcatcctg cagctgtgtt tgtgcctcct tcgctcccat tgcaaccatg caactcccac 3420
tcaactctac tcagctgtca gcacttgctg atgtcatgcc tgtttataca aataatttga 3480
ttgtttagca agcacttaga aataaaagct tcaaaagcgg aaagtatgat agctgatata 3540
ggattagagt tataggaggg gttgggtcga tttacggata atggttaacc ttgaatttgt 3600
ttgtgaaatc cgcattagct ggtatcgaaa ttggaggagg tgtttgaata gcaattgtat 3660
ttccaacgag atcaatatac aaatcacata caaactgtag gcattgaatg atcaggtgca 3720
tcgtatgata tattgagatg tcagtcagtt ctgaattata tattatttat tagtatatat 3780
atatgtactt atttcactac agttagctta gaagctattt tgctactgtg tattaatgtt 3840
catatcaaat tgccgattga aatatattca ggataaacta gatttctctt aagttaaatc 3900
aattaaatat ttctttaaat tacatctatt cacttatgaa tcaatttttg gctgtagagc 3960
aaagtgctac ctgtagattt tcaatgcacc ctttcctgtg taaattattt aaatttaatg 4020
cgtaataaat gcgaaataaa aggaaagttg cctgatgaaa aggaaacaag aaccacaatt 4080
gaaacttgag caaacaaagg gctgcggaaa atgtttgcaa attgagtttc aagtaaatat 4140
gagcaatgca atgtgaaaag taaatacacg gagagaaacg cacaccaata agtcgctgag 4200
agcgcacaac tataaacgaa gtgctgcact cacagatggt tgaaccgtgg aaaagtctct 4260
cagcttgtat ctttggtctg tgtgtgagtt tgtgtctgtg agcatctttg tatctatcgg 4320
atgcagtttt acgctttaaa aggaattctg tagtttatgc atgcgatgcg ttgtctatat 4380

aaagtttttt tccccagcac cccccctttg ccatcatctg ctgctgtctc tttcgctcgc 4440
cactaggccc tctctttcta tgactgtact ctggcagcca tggaaacgac gtcatcgcat 4500
catcgtcgtg cgttgagatg ccgaacctag ccagcaatgg cggcctcctc tccaatttca 4560
cgttccacct ctctgtcgcc ggaaaaatgc aaccaaattg ggaatcaata tgggcaaata 4620
ttgaatcata atagttcaaa tcagatttat tcattaagtt ctaagtgaac gtacaaaata 4680
gaacggtagg gaatggaggg aatggataga ggatagttta agttcctcca gagtagggga 4740
cagaaatttt tcccataaga actagacgca gcaacaaagc aagttctagt acttcagccc 4800
cccttctccc acttcaagtc catcttcttg gctctgttgt gtatctgtgt atccatggaa 4860
tacgatgtct tcctcttcat cgttttttgc cacttgcatc cgcaaaaaac tcgaaatagc 4920
catgggagat gaggtgacgg gactcataag cagcttaagg cactattaaa gccttctctc 4980
cttcttttat tttgagtcaa agcatttcaa ttaaaacata ttacgcatac gccacgttgc 5040
acgttagtga gatagaagag gattaggggg cgggcttaag agctaaaaat tcatcagaga 5100
atgttattaa agatactaaa gaatgtaaaa tgtgtctgat gcttggactc ttagaaaaag 5160
taaagcggtt aatgtatata ctaattataa aaagagaaat atgtcacatg aacgataatt 5220
ctatcgaaat tgctgtagaa gcttgcagcc attacatttg ccattaaagc atgtgtactt 5280
aaatgtgttt agttcaataa cacaacgtta tgagatccta taatccggca aacacagacc 5340
ctcaagtgac tttgctgatg tcacaaattg ctggtcagaa ttgcgtatac gccatgttga 5400
ctttcacaag ctgccacact aaatgcacct gttcaaagct cggtctcctg ctgtgtcatc 5460
aataactaac gaccataatc ctggtcaccc gcggctgctt ctgtcccttg ttccctttcc 5520
ttagttcttc ccatcattgc acattttttg ttgccaagca attgaaacgg agataaattc 5580
agagcggtag attgactgca acggagggtc gacagcagat taacccctgg cgaactgctt 5640
gattgacttt gttgcgcatg tgcaggagga tcaccctctc gaaaaaaggt tccgaacttg 5700
ccccataact gtacttgtgc aaaaagaaac taaaccgtat tcagagcatg accatcaaac 5760
aaaaggggga atcctcatcg caagatagag gggcaataca aagaagaact aagaaagtga 5820
acgagagtca gaggggaaca tatagagatt gcattccagg tcgtataaag ggttagcttt 5880
gtctacgttg caattttcaa aacatttcca tatgtacgtt tttattttct gctgctgtcg 5940
ctggtctcgt tcaaatgcat cgtaatgaat gtcatccgtt tcactttgaa ttatgtcatc 6000
cactgtgaca cacatttgca aacactacca gctcggatac acacacccat gtgcaatata 6060
taaagtcgta tatatataca atcatatatg taaatataaa catgcatagc ccaaccaact 6120
gtttctcatt agccatagaa aatgacccct tccttcc 6157

<210> 2
<211> 6157
<212> RNA
<213> Drosophila melanogaster

<220>
<221> misc_feature
<223> let7 lcnRNA

<400> 2
uuugcaaacg uaucguugca cacggcgguu cgcgaaucgc gcuuccucuu cccgcugcag 60
cugugugcgu guuguggugu acucguauug gcagcgcgua ugugugugcg uaacuacacu 120
gcaaauugug acggauucuu guguuccuca gggggaaaua acuugcguuu gacaggugcg 180
aucuagugug ccgucucaaa aaugaaaaaa aacgaugucu gguugcauuc ucuauuugcg 240
ccacugguaa gcgagaaaau ugauuaaaau gcacauuaca aagggggaaa uauauuuuaa 300
aaaaaauaug uagcuuaaau cuuaaguauu cccgugugug ugugugugug ugugugugac 360
agagagaacg agaaggagag agagagagaa cgagagagug cagccagaaa uugcaauaca 420
cacaaacaaa uugccacgca aaaagcggcc ggcuugcccu cccccuuccc uccgccauug 480
cugccccccc cuucguccau cccuuuguau gugugcauug gaugcacuug gugcgauuca 540
uuugcuuuua uuaccauuuu ugcccgucuc uccacucuuu uuuuucuuau uuuguggggc 600

cuucugcgcg auggcaaccc uggcagcaaa augcguuggu gaauaaguuc ucuacccuuu 660
cgucagugug ggacgcgacu uugaaaugcu uggagcuggc ggcaaugcag cgcuauaugc 720
aauaaaaacg uuuauaaaua aaaaaauaau auuuacugaa aucgaagaac uucccaaucg 780
gcuaaacuuu cgcaacgcuu ugaaacggcg aucggacaaa agaaaaaaau uaagaaaaug 840
ugcgugaaau guaaccaacg auuuacaaca auaguaacac uuauaauaac aaauuaauaa 900
aacaaucaau aaauauauga caaacuuguu cguuuuguga guucuccaua ccuuucuaau 960
ggauguaaua ggucugggaa guccuuuaaa aaauguauaa aagucuauau uugaaaugcu 1020
gaacuaacua uuuaguagug cauuuaaauc gaugcauuuc agacaaaacu uguacaacua 1080
cauauuccag uguuuuuacc uacacuuaaa uuauuaaauu guaaggaauc guguuuuugg 1140
gcacguuuuu agucgauuuc agggcugagc aauuuuuaaa accuuuuucu auuuaggaua 1200
uaaaaucaaa agaguuuccc ucuacaauuu ucaugccgaa aaccaauuga aggcaauuaa 1260
aacgcauuuu cugugcaauu gaugaugacc uacgacaaag gaaaugaagu cauacccaua 1320
cacacacgac ucucacgcac acauccacaa agcuagaggc aaagugcagg ccaaaagcga 1380
augaagaaca aaaacaacaa gcaggggccc uacgaaagcc aacaacaaca aagugcaaau 1440
ugcacugcgc augcacaugc acaaacacac acacacacag gcaaccgcag cacacacaga 1500
uacuccaacu gaggcgaauu gcaguggugu ucgggcgcuu uuguucgccu gcgaaaugaa 1560
aaaacagcuc gaaaaaguac uuaaaaauau auuuaaauuu uaaaaauuac ucgaaaggcu 1620
auuccaaaau auucacaaaa uuggggaacu uuaauaaauu ucaauuuuuu ccgccugcau 1680
uccuccucuu ucuggcuucc uuuauuuaua auauuaaaau gcgcgucaug ccaacgacag 1740
uugaaugcac ucggccuuug ccuucaaugg cuucccccuu gacugauaau gauaauaaac 1800
agauuauucg cuuuuuccaa uacguuuuug uauuaaaagc aagcucucca gccgcuagcu 1860
ucacuuugau gccacaaagc augcuucuua agcaguuuuc gauauuugag aguaaaaauc 1920
aacucuuuuu uucaauauuu cuagcuguuc guuucaaagc uguuuggaug ucauugaauu 1980
ucaauacuua cugaauacgc guugauauuc uauugucuug acaauaucua cauuuaugaa 2040
uccaucucca aauauguaau acguuaaaca guauauucuu auccugacag aguugcauag 2100
uccacauuuu auacgcuuac uccgauauua uuuauuuagu ucugacucau aguccgacgg 2160
cguuuccuuu cgaccuugag ugaaguauuu aaaguagaau gggcagugca auuauuaaau 2220
ucucugcgaa ccgcagagaa uugcacuugg ugguugucgu ucucugggcc gcuggcuuga 2280
guuguguauc uacaacuaga auuacucauc aucagcauuu ccauagaaau cagaacucaa 2340
aacaauaaac caaaguaagg aaggccguuu aauuauuaaa uagaaggcuu uuauacaaca 2400
uuuacuuacu uaaucugguu aaaaacugca gugaaaacua auuuuuaaaa aacaaaacuu 2460
uuuauaacua uaauuguuaa aagagauuuu aaaaugaaaa cuacauaaca gaaaaagcga 2520
uuaaacuuua uauauaaaug auaucuaaca aaggaccuaa aaggaccaua aacaggaaug 2580
cuaacaaaca uaaaaucaaa guaaauguga auuuauagaa uccuuucagg acuuaaaaac 2640
uuugcaagca aaacggcaaa aauauacaag aaaugacagu acuuuuccua cuuccuucuu 2700
ucguaccauu ugaacuccau uuuuuuuguu gggcgagugc aaaagggaug gguagaggug 2760
ccaaaggaca augguucaag ugcaaagagg augccaagcc gcaugcaaca augccaacaa 2820
caaggaccga acgaacgccg aaggacaugc cgugcaauuu ccuuuucugu ggugaguugc 2880
agcuauugcg uauacgccgu gugguguaca gagcgaaaga gaggccacua ugccgucugg 2940
auagacagag agcgacgcua uuuauagcag ggagcuccaa aaccgagcuc agccuugauu 3000
ccgcccuuga acuaacauug cuggggaacu auuccugucu cuccaguccu gacccuuucc 3060
gucccaaccc guccauuccc gauuucguac ccuucgaauc gcccagcagu cgucacagcu 3120
ggaaaccagu ugggggagca cauaugagga ggaugugcca ggggaauacu ggagguacag 3180
gagguacuug gggcaucgag aaccacagug cauaugcugc cagucaagcg guaauuuuga 3240
guuugcaguu cgcucuuucg cacuguccca ugccucaugc cccaugcucc augccccaug 3300
cuccaugucc cauguccuug ccccaccaaa cugaacucag caggcuuuga cauugauuuu 3360
gugcauccug cagcuguguu ugugccuccu ucgcucccau ugcaaccaug caacucccac 3420
ucaacucuac ucagcuguca gcacuugcug augucaugcc uguuuauaca aauaauuuga 3480
uuguuuagca agcacuuaga aauaaaagcu ucaaaagcgg aaaguaugau agcugauaua 3540
ggauuagagu uauaggaggg guugggucga uuuacggaua augguuaacc uugaauuugu 3600
uugugaaauc cgcauuagcu gguaucgaaa uuggaggagg uguuugaaua gcaauuguau 3660
uuccaacgag aucaauauac aaaucacaua caaacuguag gcauugaaug aucaggugca 3720
ucguaugaua uauugagaug ucagucaguu cugaauuaua uauuauuuau uaguauauau 3780

auauguacuu auuucacuac aguuagcuua gaagcuauuu ugcuacugug uauuaauguu 3840
cauaucaaau ugccgauuga aauauauuca ggauaaacua gauuucucuu aaguuaaauc 3900
aauuaaauau uucuuuaaau uacaucuauu cacuuaugaa ucaauuuuug gcuguagagc 3960
aaagugcuac cuguagauuu ucaaugcacc cuuuccugug uaaauuauuu aaauuuaaug 4020
cguaauaaau gcgaaauaaa aggaaaguug ccugaugaaa aggaaacaag aaccacaauu 4080
gaaacuugag caaacaaagg gcugcggaaa auguuugcaa auugaguuuc aaguaaauau 4140
gagcaaugca augugaaaag uaaauacacg gagagaaacg cacaccaaua agucgcugag 4200
agcgcacaac uauaaacgaa gugcugcacu cacagauggu ugaaccgugg aaaagucucu 4260
cagcuuguau cuuuggucug ugugugaguu ugugucugug agcaucuuug uaucuaucgg 4320
augcaguuuu acgcuuuaaa aggaauucug uaguuuaugc augcgaugcg uugucuauau 4380
aaaguuuuuu uccccagcac ccccccuuug ccaucaucug cugcugucuc uuucgcucgc 4440
cacuaggccc ucucuuucua ugacuguacu cuggcagcca uggaaacgac gucaucgcau 4500
caucgucgug cguugagaug ccgaaccuag ccagcaaugg cggccuccuc uccaauuuca 4560
cguuccaccu cucugucgcc ggaaaaaugc aaccaaauug ggaaucaaua ugggcaaaua 4620
uugaaucaua auaguucaaa ucagauuuau ucauuaaguu cuaagugaac guacaaaaua 4680
gaacgguagg gaauggaggg aauggauaga ggauaguuua aguuccucca gaguagggga 4740
cagaaauuuu ucccauaaga acuagacgca gcaacaaagc aaguucuagu acuucagccc 4800
cccuucuccc acuucaaguc caucuucuug gcucuguugu guaucugugu auccauggaa 4860
uacgaugucu uccucuucau cguuuuuugc cacuugcauc cgcaaaaaac ucgaaauagc 4920
caugggagau gaggugacgg gacucauaag cagcuuaagg cacuauuaaa gccuucucuc 4980
cuucuuuuau uuugagucaa agcauuucaa uuaaaacaua uuacgcauac gccacguugc 5040
acguuaguga gauagaagag gauuaggggg cgggcuuaag agcuaaaaau ucaucagaga 5100
auguuauuaa agauacuaaa gaauguaaaa ugugucugau gcuuggacuc uuagaaaaag 5160
uaaagcgguu aauguauaua cuaauuauaa aaagagaaau augucacaug aacgauaauu 5220
cuaucgaaau ugcuguagaa gcuugcagcc auuacauuug ccauuaaagc auguguacuu 5280
aaauguguuu aguucaauaa cacaacguua ugagauccua uaauccggca aacacagacc 5340
cucaagugac uuugcugaug ucacaaauug cuggucagaa uugcguauac gccauguuga 5400
cuuucacaag cugccacacu aaaugcaccu guucaaagcu cggucuccug cugugucauc 5460
aauaacuaac gaccauaauc cuggucaccc gcggcugcuu cugucccuug uucccuuucc 5520
uuaguucuuc ccaucauugc acauuuuuug uugccaagca auugaaacgg agauaaauuc 5580
agagcgguag auugacugca acggaggguc gacagcagau uaaccccugg cgaacugcuu 5640
gauugacuuu guugcgcaug ugcaggagga ucacccucuc gaaaaaaggu uccgaacuug 5700
ccccauaacu guacuugugc aaaaagaaac uaaaccguau ucagagcaug accaucaaac 5760
aaaaggggga auccucaucg caagauagag gggcaauaca aagaagaacu aagaaaguga 5820
acgagaguca gaggggaaca uauagagauu gcauuccagg ucguauaaag gguuagcuuu 5880
gucuacguug caauuuucaa aacauuucca uauguacguu uuuauuuucu gcugcugucg 5940
cuggucucgu ucaaaugcau cguaaugaau gucauccguu ucacuuugaa uuaugucauc 6000
cacugugaca cacauuugca aacacuacca gcucggauac acacacccau gugcaauaua 6060
uaaagucgua uauauauaca aucauauaug uaaauauaaa caugcauagc ccaaccaacu 6120
guuucucauu agccauagaa aaugaccccu uccuucc 6157

SEQUENCE LISTING

<110>  ETH Zurich

<120>  RNA molecules for the treatment of cancer

<130>  38407-ETH-P-EP1

<150>  EP 20177607.7
<151>  2020-05-29

<160>  2

<170>  PatentIn version 3.5

<210>  1
<211>  6157
<212>  DNA
<213>  Drosophila melanogaster

<220>
<221>  misc_feature
<223>  Drosophila melanogaster BDGP6.28, Chromosome 2L:
       18,455,718-18,461,874

<400>  1
tttgcaaacg tatcgttgca cacggcggtt cgcgaatcgc gcttcctctt cccgctgcag      60

ctgtgtgcgt gttgtggtgt actcgtattg gcagcgcgta tgtgtgtgcg taactacact     120

gcaaattgtg acggattctt gtgttcctca gggggaaata acttgcgttt gacaggtgcg     180

atctagtgtg ccgtctcaaa aatgaaaaaa aacgatgtct ggttgcattc tctatttgcg     240

ccactggtaa gcgagaaaat tgattaaaat gcacattaca aagggggaaa tatattttaa     300

aaaaaatatg tagcttaaat cttaagtatt cccgtgtgtg tgtgtgtgtg tgtgtgtgac     360

agagagaacg agaaggagag agagagagaa cgagagagtg cagccagaaa ttgcaataca     420

cacaaacaaa ttgccacgca aaaagcggcc ggcttgccct cccccttccc tccgccattg     480

ctgccccccc cttcgtccat ccctttgtat gtgtgcattg gatgcacttg gtgcgattca     540

tttgctttta ttaccatttt tgcccgtctc tccactcttt tttttcttat tttgtggggc     600

cttctgcgcg atggcaaccc tggcagcaaa atgcgttggt gaataagttc tctacccttt     660

cgtcagtgtg ggacgcgact ttgaaatgct tggagctggc ggcaatgcag cgctatatgc     720

aataaaaacg tttataaata aaaaaataat atttactgaa atcgaagaac ttcccaatcg     780

gctaaacttt cgcaacgctt tgaaacggcg atcggacaaa agaaaaaaat taagaaaatg     840

tgcgtgaaat gtaaccaacg atttacaaca atagtaacac ttataataac aaattaataa     900

aacaatcaat aaatatatga caaacttgtt cgttttgtga gttctccata cctttctaat     960

ggatgtaata ggtctgggaa gtcctttaaa aaatgtataa aagtctatat ttgaaatgct    1020

gaactaacta tttagtagtg catttaaatc gatgcatttc agacaaaact tgtacaacta    1080

catattccag tgtttttacc tacacttaaa ttattaaatt gtaaggaatc gtgtttttgg 1140

gcacgttttt agtcgatttc agggctgagc aatttttaaa acctttttct atttaggata 1200

taaaatcaaa agagtttccc tctacaattt tcatgccgaa aaccaattga aggcaattaa 1260

aacgcatttt ctgtgcaatt gatgatgacc tacgacaaag gaaatgaagt catacccata 1320

cacacacgac tctcacgcac acatccacaa agctagaggc aaagtgcagg ccaaaagcga 1380

atgaagaaca aaaacaacaa gcaggggccc tacgaaagcc aacaacaaca aagtgcaaat 1440

tgcactgcgc atgcacatgc acaaacacac acacacacag gcaaccgcag cacacacaga 1500

tactccaact gaggcgaatt gcagtggtgt tcgggcgctt ttgttcgcct gcgaaatgaa 1560

aaaacagctc gaaaaagtac ttaaaaatat atttaaattt taaaaattac tcgaaaggct 1620

attccaaaat attcacaaaa ttggggaact ttaataaatt tcaatttttt ccgcctgcat 1680

tcctcctctt tctggcttcc tttatttata atattaaaat gcgcgtcatg ccaacgacag 1740

ttgaatgcac tcggcctttg ccttcaatgg cttccccctt gactgataat gataataaac 1800

agattattcg ctttttccaa tacgtttttg tattaaaagc aagctctcca gccgctagct 1860

tcactttgat gccacaaagc atgcttctta agcagttttc gatatttgag agtaaaaatc 1920

aactcttttt ttcaatattt ctagctgttc gtttcaaagc tgtttggatg tcattgaatt 1980

tcaatactta ctgaatacgc gttgatattc tattgtcttg acaatatcta catttatgaa 2040

tccatctcca aatatgtaat acgttaaaca gtatattctt atcctgacag agttgcatag 2100

tccacatttt atacgcttac tccgatatta tttatttagt tctgactcat agtccgacgg 2160

cgtttccttt cgaccttgag tgaagtattt aaagtagaat gggcagtgca attattaaat 2220

tctctgcgaa ccgcagagaa ttgcacttgg tggttgtcgt tctctgggcc gctggcttga 2280

gttgtgtatc tacaactaga attactcatc atcagcattt ccatagaaat cagaactcaa 2340

aacaataaac caaagtaagg aaggccgttt aattattaaa tagaaggctt ttatacaaca 2400

tttacttact taatctggtt aaaaactgca gtgaaaacta atttttaaaa aacaaaactt 2460

tttataacta taattgttaa aagagatttt aaaatgaaaa ctacataaca gaaaaagcga 2520

ttaaacttta tatataaatg atatctaaca aaggacctaa aaggaccata aacaggaatg 2580

ctaacaaaca taaaatcaaa gtaaatgtga atttatagaa tcctttcagg acttaaaaac 2640

tttgcaagca aaacggcaaa aatatacaag aaatgacagt acttttccta cttccttctt 2700

tcgtaccatt tgaactccat tttttttgtt gggcgagtgc aaaagggatg ggtagaggtg 2760

ccaaaggaca atggttcaag tgcaaagagg atgccaagcc gcatgcaaca atgccaacaa 2820

caaggaccga acgaacgccg aaggacatgc cgtgcaattt ccttttctgt ggtgagttgc 2880

agctattgcg tatacgccgt gtggtgtaca gagcgaaaga gaggccacta tgccgtctgg 2940

atagacagag agcgacgcta tttatagcag ggagctccaa aaccgagctc agccttgatt 3000

ccgcccttga actaacattg ctggggaact attcctgtct ctccagtcct gaccctttcc 3060

gtcccaaccc gtccattccc gatttcgtac ccttcgaatc gcccagcagt cgtcacagct 3120

ggaaaccagt tgggggagca catatgagga ggatgtgcca ggggaatact ggaggtacag 3180

gaggtacttg gggcatcgag aaccacagtg catatgctgc cagtcaagcg gtaattttga 3240

gtttgcagtt cgctctttcg cactgtccca tgcctcatgc cccatgctcc atgccccatg 3300

ctccatgtcc catgtccttg ccccaccaaa ctgaactcag caggctttga cattgatttt 3360

gtgcatcctg cagctgtgtt tgtgcctcct tcgctcccat tgcaaccatg caactcccac 3420

tcaactctac tcagctgtca gcacttgctg atgtcatgcc tgtttataca aataatttga 3480

ttgtttagca agcacttaga aataaaagct tcaaaagcgg aaagtatgat agctgatata 3540

ggattagagt tataggaggg gttgggtcga tttacggata atggttaacc ttgaatttgt 3600

ttgtgaaatc cgcattagct ggtatcgaaa ttggaggagg tgtttgaata gcaattgtat 3660

ttccaacgag atcaatatac aaatcacata caaactgtag gcattgaatg atcaggtgca 3720

tcgtatgata tattgagatg tcagtcagtt ctgaattata tattatttat tagtatatat 3780

atatgtactt atttcactac agttagctta gaagctattt tgctactgtg tattaatgtt 3840

catatcaaat tgccgattga aatatattca ggataaacta gatttctctt aagttaaatc 3900

aattaaatat ttctttaaat tacatctatt cacttatgaa tcaatttttg gctgtagagc 3960

aaagtgctac ctgtagattt tcaatgcacc ctttcctgtg taaattattt aaatttaatg 4020

cgtaataaat gcgaaataaa aggaaagttg cctgatgaaa aggaaacaag aaccacaatt 4080

gaaacttgag caaacaaagg gctgcggaaa atgtttgcaa attgagtttc aagtaaatat 4140

gagcaatgca atgtgaaaag taaatacacg gagagaaacg cacaccaata agtcgctgag 4200

agcgcacaac tataaacgaa gtgctgcact cacagatggt tgaaccgtgg aaaagtctct 4260

cagcttgtat ctttggtctg tgtgtgagtt tgtgtctgtg agcatctttg tatctatcgg 4320

atgcagtttt acgctttaaa aggaattctg tagtttatgc atgcgatgcg ttgtctatat 4380

aaagtttttt tccccagcac cccccctttg ccatcatctg ctgctgtctc tttcgctcgc 4440

cactaggccc tctctttcta tgactgtact ctggcagcca tggaaacgac gtcatcgcat 4500

catcgtcgtg cgttgagatg ccgaacctag ccagcaatgg cggcctcctc tccaatttca 4560

cgttccacct ctctgtcgcc ggaaaaatgc aaccaaattg ggaatcaata tgggcaaata 4620

ttgaatcata atagttcaaa tcagatttat tcattaagtt ctaagtgaac gtacaaaata 4680

gaacggtagg gaatggaggg aatggataga ggatagttta agttcctcca gagtagggga 4740

cagaaatttt tcccataaga actagacgca gcaacaaagc aagttctagt acttcagccc 4800

cccttctccc acttcaagtc catcttcttg gctctgttgt gtatctgtgt atccatggaa 4860

tacgatgtct tcctcttcat cgttttttgc cacttgcatc cgcaaaaaac tcgaaatagc 4920

catgggagat gaggtgacgg gactcataag cagcttaagg cactattaaa gccttctctc 4980

cttcttttat tttgagtcaa agcatttcaa ttaaaacata ttacgcatac gccacgttgc 5040

acgttagtga gatagaagag gattaggggg cgggcttaag agctaaaaat tcatcagaga 5100

atgttattaa agatactaaa gaatgtaaaa tgtgtctgat gcttggactc ttagaaaaag 5160

taaagcggtt aatgtatata ctaattataa aaagagaaat atgtcacatg aacgataatt 5220

ctatcgaaat tgctgtagaa gcttgcagcc attacatttg ccattaaagc atgtgtactt 5280

aaatgtgttt agttcaataa cacaacgtta tgagatccta taatccggca aacacagacc 5340

ctcaagtgac tttgctgatg tcacaaattg ctggtcagaa ttgcgtatac gccatgttga 5400

ctttcacaag ctgccacact aaatgcacct gttcaaagct cggtctcctg ctgtgtcatc 5460

aataactaac gaccataatc ctggtcaccc gcggctgctt ctgtcccttg ttccctttcc 5520

ttagttcttc ccatcattgc acattttttg ttgccaagca attgaaacgg agataaattc 5580

agagcggtag attgactgca acggagggtc gacagcagat taacccctgg cgaactgctt 5640

gattgacttt gttgcgcatg tgcaggagga tcaccctctc gaaaaaaggt tccgaacttg 5700

ccccataact gtacttgtgc aaaaagaaac taaaccgtat tcagagcatg accatcaaac 5760

aaaaggggga atcctcatcg caagatagag gggcaataca aagaagaact aagaaagtga 5820

acgagagtca gaggggaaca tatagagatt gcattccagg tcgtataaag ggttagcttt 5880

gtctacgttg caattttcaa aacatttcca tatgtacgtt tttattttct gctgctgtcg 5940

ctggtctcgt tcaaatgcat cgtaatgaat gtcatccgtt tcactttgaa ttatgtcatc 6000

cactgtgaca cacatttgca aacactacca gctcggatac acacacccat gtgcaatata 6060

taaagtcgta tatatataca atcatatatg taaatataaa catgcatagc ccaaccaact 6120

gtttctcatt agccatagaa aatgacccct tccttcc 6157

<210> 2
<211> 6157
<212> RNA
<213> Drosophila melanogaster

<220>
<221> misc_feature
<223> let7 lcnRNA

<400> 2
uuugcaaacg uaucguugca cacggcgguu cgcgaaucgc gcuuccucuu cccgcugcag 60

cugugugcgu guuguggugu acucguauug gcagcgcgua ugugugugcg uaacuacacu 120

gcaaauugug acggauucuu guguuccuca gggggaaaua acuugcguuu gacaggugcg 180

aucuagugug ccgucucaaa aaugaaaaaa aacgaugucu gguugcauuc ucuauuugcg 240

ccacugguaa gcgagaaaau ugauuaaaau gcacauuaca aagggggaaa uauauuuuaa 300

aaaaaauaug uagcuuaaau cuuaaguauu cccgugugug ugugugugug ugugugugac 360

agagagaacg agaaggagag agagagagaa cgagagagug cagccagaaa uugcaauaca 420

cacaaacaaa uugccacgca aaaagcggcc ggcuugcccu cccccuuccc uccgccauug 480

cugccccccc cuucguccau cccuuuguau gugugcauug gaugcacuug gugcgauuca 540

uuugcuuuua uuaccauuuu ugcccgucuc uccacucuuu uuuuucuuau uuuguggggc 600

cuucugcgcg auggcaaccc uggcagcaaa augcguuggu gaauaaguuc ucuacccuuu 660

cgucagugug ggacgcgacu uugaaaugcu uggagcuggc ggcaaugcag cgcuauaugc 720

aauaaaaacg uuuauaaaua aaaaaauaau auuuacugaa aucgaagaac uucccaaucg 780

gcuaaacuuu cgcaacgcuu ugaaacggcg aucggacaaa agaaaaaaau uaagaaaaug 840

ugcgugaaau guaaccaacg auuuacaaca auaguaacac uuauaauaac aaauuaauaa 900

aacaaucaau aaauauauga caaacuuguu cguuuuguga guucuccaua ccuuucuaau 960

ggauguaaua ggucugggaa guccuuuaaa aaauguauaa aagucuauau uugaaaugcu 1020

gaacuaacua uuuaguagug cauuuaaauc gaugcauuuc agacaaaacu uguacaacua 1080

cauauuccag uguuuuuacc uacacuuaaa uuauuaaauu guaaggaauc guguuuuugg 1140

gcacguuuuu agucgauuuc agggcugagc aauuuuuaaa accuuuuucu auuuaggaua 1200

uaaaaucaaa agaguuuccc ucuacaauuu ucaugccgaa aaccaauuga aggcaauuaa 1260

aacgcauuuu cugugcaauu gaugaugacc uacgacaaag gaaaugaagu cauacccaua 1320

cacacacgac ucucacgcac acauccacaa agcuagaggc aaagugcagg ccaaaagcga 1380

augaagaaca aaaacaacaa gcaggggccc uacgaaagcc aacaacaaca aagugcaaau 1440

ugcacugcgc augcacaugc acaaacacac acacacacag gcaaccgcag cacacacaga 1500

uacuccaacu gaggcgaauu gcaguggugu ucgggcgcuu uuguucgccu gcgaaaugaa 1560

aaaacagcuc gaaaaaguac uuaaaaauau auuuaaauuu uaaaaauuac ucgaaaggcu 1620

auuccaaaau auucacaaaa uuggggaacu uuaauaaauu ucaauuuuuu ccgccugcau 1680

uccuccucuu ucuggcuucc uuuauuuaua auauuaaaau gcgcgucaug ccaacgacag 1740

uugaaugcac ucggccuuug ccuucaaugg cuucccccuu gacugauaau gauaauaaac 1800

agauuauucg cuuuuuccaa uacguuuuug uauuaaaagc aagcucucca gccgcuagcu 1860

ucacuuugau gccacaaagc augcuucuua agcaguuuuc gauauuugag aguaaaaauc 1920

aacucuuuuu uucaauauuu cuagcuguuc guuucaaagc uguuuggaug ucauugaauu 1980

ucaauacuua cugaauacgc guugauauuc uauugucuug acaauaucua cauuuaugaa 2040

uccaucucca aauauguaau acguuaaaca guauauucuu auccugacag aguugcauag 2100

uccacauuuu auacgcuuac uccgauauua uuuauuuagu ucugacucau aguccgacgg 2160

cguuuccuuu cgaccuugag ugaaguauuu aaaguagaau gggcagugca auuauuaaau 2220

ucucugcgaa ccgcagagaa uugcacuugg ugguugucgu ucucugggcc gcuggcuuga 2280

guuguguauc uacaacuaga auuacucauc aucagcauuu ccauagaaau cagaacucaa 2340

aacaauaaac caaaguaagg aaggccguuu aauuauuaaa uagaaggcuu uuauacaaca 2400

uuuacuuacu uaaucugguu aaaaacugca gugaaacuaa auuuuaaaaa aacaaaacuu 2460

uuuauaacua uaauuguuaa aagagauuuu aaaaugaaaa cuacauaaca gaaaaagcga 2520

uuaaacuuua uauauaaaug auaucuaaca aaggaccuaa aaggaccaua aacaggaaug 2580

cuaacaaaca uaaaaucaaa guaaauguga auuuauagaa uccuuucagg acuuaaaaac 2640

uuugcaagca aaacggcaaa aauauacaag aaaugacagu acuuuuccua cuuccuucuu 2700

ucguaccauu ugaacuccau uuuuuuuguu gggcgagugc aaaagggaug gguagaggug 2760

ccaaaggaca augguucaag ugcaaagagg augccaagcc gcaugcaaca augccaacaa 2820

caaggaccga acgaacgccg aaggacaugc cgugcaauuu ccuuuucugu ggugaguugc 2880

agcuauugcg uauacgccgu gugguguaca gagcgaaaga gaggccacua ugccgucugg 2940

auagacagag agcgacgcua uuuauagcag ggagcuccaa aaccgagcuc agccuugauu 3000

ccgcccuuga acuaacauug cuggggaacu auuccugucu cuccaguccu gacccuuucc 3060

gucccaaccc guccauuccc gauuucguac ccuucgaauc gcccagcagu cgucacagcu 3120

ggaaaccagu ugggggagca cauaugagga ggaugugcca ggggaauacu ggagguacag 3180

gagguacuug gggcaucgag aaccacagug cauaugcugc cagucaagcg guaauuuuga 3240

guuugcaguu cgcucuuucg cacuguccca ugccucaugc cccaugcucc augccccaug 3300

cuccaugucc cauguccuug ccccaccaaa cugaacucag caggcuuuga cauugauuuu 3360

gugcauccug cagcuguguu ugugccuccu ucgcucccau ugcaaccaug caacucccac 3420

ucaacucuac ucagcuguca gcacuugcug augucaugcc uguuuauaca aauaauuuga 3480

uuguuuagca agcacuuaga aauaaaagcu ucaaaagcgg aaaguaugau agcugauaua 3540

ggauuagagu uauaggaggg guuggucgau uuacggauaa augguuaacc uugaauuugu 3600

uugugaaauc cgcauuagcu gguaucgaaa uuggaggagg uguuugaaua gcaauuguau 3660

uuccaacgag aucaauauac aaaucacaua caaacuguag gcauugaaug aucaggugca 3720

ucguaugaua uauugagaug ucagucaguu cugaauuaua uauuauuuau uaguauauau 3780

auauguacuu auuucacuac aguuagcuua gaagcuauuu ugcuacugug uauuaauguu 3840

cauaucaaau ugccgauuga aauauauuca ggauaaacua gauuucucuu aaguuaaauc 3900

aauuaaauau uucuuuaaau uacaucuauu cacuuaugaa ucaauuuuug gcuguagagc 3960

aaagugcuac cuguagauuu ucaaugcacc cuuuccugug uaaauuauuu aaauuuaaug 4020

cguaauaaau gcgaaauaaa aggaaaguug ccugaugaaa aggaaacaag aaccacaauu 4080

gaaacuugag caaacaaagg gcugcggaaa auguuugcaa auugaguuuc aaguaaauau 4140

gagcaaugca augugaaaag uaaauacacg gagagaaacg cacaccaaua agucgcugag 4200

agcgcacaac uauaaacgaa gugcugcacu cacagauggu ugaaccgugg aaaagucucu 4260

cagcuuguau cuuuggucug ugugugaguu ugugucugug agcaucuuug uaucuaucgg 4320

augcaguuuu acgcuuuaaa aggaauucug uaguuuaugc augcgaugcg uugucuauau 4380

aaaguuuuuu uccccagcac ccccccuuug ccaucaucug cugcugucuc uuucgcucgc 4440

cacuaggccc ucucuuucua ugacuguacu cuggcagcca uggaaacgac gucaucgcau 4500

caucgucgug cguugagaug ccgaaccuag ccagcaaugg cggccuccuc uccaauuuca 4560

cguuccaccu cucugucgcc ggaaaaaugc aaccaaauug ggaaucaaua ugggcaaaua 4620

uugaaucaua auaguucaaa ucagauuuau ucauuaaguu cuaagugaac guacaaaaua 4680

gaacgguagg gaauggaggg aauggauaga ggauaguuua aguuccucca gaguagggga 4740

cagaaauuuu ucccauaaga acuagacgca gcaacaaagc aaguucuagu acuucagccc 4800

cccuucuccc acuucaaguc caucuucuug gcucuguugu guaucugugu auccauggaa 4860

uacgaugucu uccucuucau cguuuuuugc cacuugcauc cgcaaaaaac ucgaaauagc 4920

caugggagau gaggugacgg gacucauaag cagcuuaagg cacuauuaaa gccuucucuc 4980

cuucuuuuau uuugagucaa agcauuucaa uuaaaacaua uuacgcauac gccacguugc 5040

acguuaguga gauagaagag gauuaggggg cgggcuuaag agcuaaaaau ucaucagaga 5100

auguuauuaa agauacuaaa gaauguaaaa ugugucugau gcuuggacuc uuagaaaaag 5160

uaaagcgguu aauguauaua cuaauuauaa aaagagaaau augucacaug aacgauaauu 5220

cuaucgaaau ugcuguagaa gcuugcagcc auuacauuug ccauuaaagc auguguacuu 5280

aaauguguuu aguucaauaa cacaacguua ugagauccua uaauccggca aacacagacc 5340

cucaagugac uuugcugaug ucacaaauug cuggucagaa uugcguauac gccauguuga 5400

cuuucacaag cugccacacu aaaugcaccu guucaaagcu cggucuccug cugugucauc 5460

aauaacuaac gaccauaauc cuggucaccc gcggcugcuu cugucccuug uucccuuucc 5520

uuaguucuuc ccaucauugc acauuuuuug uugccaagca auugaaacgg agauaaauuc 5580

agagcgguag auugacugca acggaggguc gacagcagau uaaccccugg cgaacugcuu 5640

gauugacuuu guugcgcaug ugcaggagga ucacccucuc gaaaaaaggu uccgaacuug 5700

ccccauaacu guacuugugc aaaaagaaac uaaaccguau ucagagcaug accaucaaac 5760

aaaaggggga auccucaucg caagauagag gggcaauaca aagaagaacu aagaaaguga 5820

acgagaguca gaggggaaca uauagagauu gcauuccagg ucguauaaag gguuagcuuu 5880

gucuacguug caauuuucaa aacauuucca uauguacguu uuuauuuucu gcugcugucg 5940

cuggucucgu ucaaaugcau cguaaugaau gucauccguu ucacuuugaa uuaugucauc 6000

cacugugaca cacauuugca aacacuacca gcucggauac acacacccau gugcaauaua 6060

uaaagucgua uauauauaca aucauauaug uaaauauaaa caugcauagc ccaaccaacu 6120

guuucucauu agccauagaa aaugaccccu uccuucc 6157

## Description

### Field of the invention

The present invention is directed to an RNA molecule having a sequence having at least 80 % sequence identity to SEQ ID NO: 2. Furthermore, the invention is directed to an RNA molecule obtainable by transcription of a DNA sequence having at least 80% sequence identity to SEQ ID NO: 1 for use in the treatment of cancer. In addition, the invention is directed to a pharmaceutical composition comprising as active ingredient a RNA molecule obtainable by transcription of a DNA sequence having at least 80% sequence identity to SEQ ID NO: 1 or a vector loaded with a DNA sequence having at least 80% sequence identity to SEQ ID NO: 1.

### Background of the invention

The large-scale sequencing and mapping of genomes uncovered the occurrence of extended transcribed regions, producing RNAs without an apparent coding potential. While many of these non-coding RNAs can be assigned to well-known functional families, like rRNAs, tRNAs, snoRNAs or miRNAs, the majority of these transcripts remain functionally uncharacterized. A particular class, long non-coding RNAs (IncRNAs), shows many hallmarks of mRNAs, like cap-structure and polyA tail or the usage of the same transcriptional and post-transcriptional machinery, except for the capacity to encode the information for a protein. There is increasing evidence, however, that IncRNAs also fulfil a broad range of cellular and organismal functions as well (Kopp, F. & Mendell, J.T. (2018). Functional classification and experimental dissection of long noncoding RNAs. Cell. 172, 393-407.). While in the past the conservation of the open reading frame in mRNAs often supported the functional characterization of the associated proteins across species, the much-reduced evolutionary preservation of IncRNA sequences hampers this task substantially. Often the function is restricted to a characteristic secondary structure allowing the interactions with highly specific partner molecules. Hence, complex research strategies are often required to reveal underlying functional roles of specific IncRNAs.

*Drosophila* is a powerful model to study the mechanisms underlying tumor formation and development (Read, R.D. (2011). Drosophila melanogaster as a model system for human brain cancers. Glia. 59(9):1364-1376; Miles, W.O., Dyson, N.J., and Walker, J.A. (2011). Modeling tumor invasion and metastasis in Drosophila. Dis Model Mech. 4(6):753-761; Gonzalez, C. (2013). Drosophila melanogaster: a model and a tool to investigate malignancy and identify new therapeutics. Nat Rev Cancer 13, 172-183.). The central brain of *Drosophila* has been used to study neural stem cell-derived brain tumors in *brain tumor* (*brat*) gene mutants (Jiang, Y. & Reichert, H. (2014). Drosophila neural stem cells in brain development and tumor formation. J Neurogenet. 28, 181-189.).

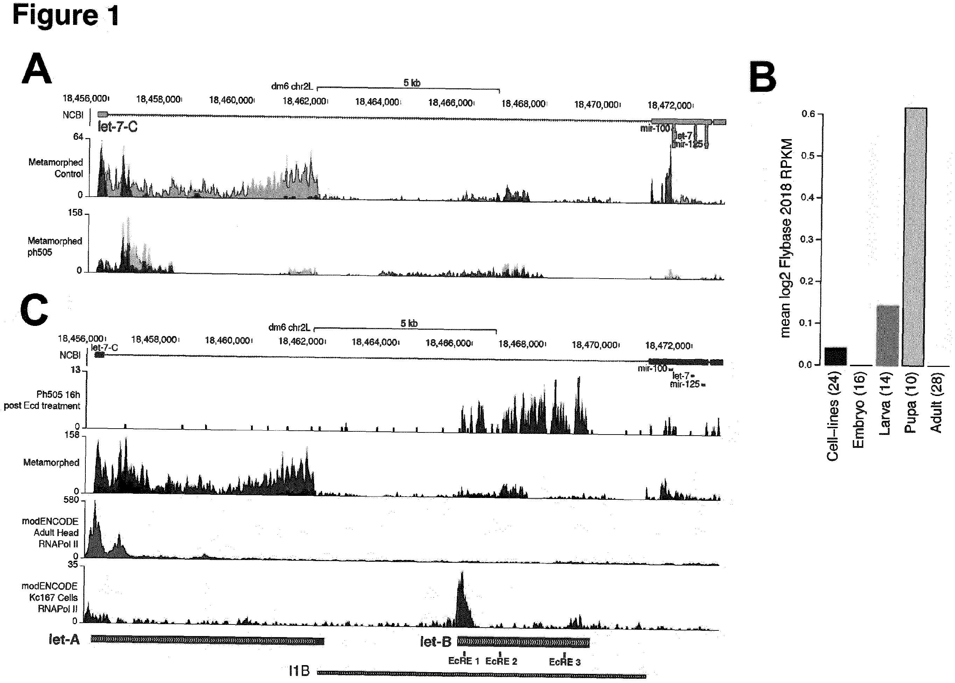

*lethal-7* (*let-7*) encodes an evolutionarily highly conserved microRNA (miRNA) and was the first miRNA to be discovered (Reinhart B.J., Slack F.J., Basson M., Pasquinelli A.E., Bettinger J.C., Rougvie A.E., Horvitz H.R., and Ruvkun G. (2000). The 21-nucleotide let-7 RNA regulates developmental timing in Caenorhabditis elegans. Nature. 403, 901-906.). In *Drosophila*, *let-7* is located in the let-7 complex (*let-7-C*), which is a locus on the second chromosome. Its primary transcript encoding besides let-7 and mir-125 also mir-100 spans -17 kb of genomic region and consisting of three exons and two introns (Fig. 1A) (Büssing, I., Slack, F.J., and Grosshans, H. (2008). let-7 microRNAs in development, stem cells and cancer. Trends Mol Med. 14, 400-409.).

During the development of *Drosophila,* expression of let-7, miR-100 and miR-125 is regulated by the steroid hormone ecdysone (Sempere, L.F., Dubrovsky, E.B., Dubrovskaya, V.A., Berger, E.M., and Ambros, V. (2002). The expression of the let-7 small regulatory RNA is controlled by ecdysone during metamorphosis in Drosophila melanogaster. Dev Biol. 244, 170-179.). Studies in *C. elegans* and *Drosophila* have shown that *let-7* is required for controlling developmental timing and for cell differentiation (Büssing, I., Slack, F.J., and Grosshans, H. (2008). let-7 microRNAs in development, stem cells and cancer. Trends Mol Med. 14, 400-409.). Although flies containing a complete deletion of all three miRNAs are viable and appear morphologically normal, the mutant adult flies display behavioral and aging defects, indicating that *let-7-C* is required for neuronal development, neuromuscular remodeling, and adult lifespan and aging (Sokol, N.S., Xu, P., Jan, Y.N., and Ambros, V. (2008). Drosophila let-7 microRNA is required for remodeling of the neuromusculature during metamorphosis. Genes Dev. 22, 1591-1596; Chawla, G., Deosthale, P., Childress, S., Wu, Y.C., and Sokol, N.S. (2016). A let-7-to-miR-125 microRNA switch regulates neuronal integrity and lifespan in Drosophila. PLoS Genet. 12(8): e1006247; Gendron, C.M. & Pletcher, S.D. (2017). MicroRNAs mir-184 and let-7 alter Drosophila metabolism and longevity. Aging Cell. 16, 1434-1438.). In mammals, all three miRNAs are jointly present as large gene families located on different chromosomes in the genome. Due to the functional redundancy and complexity, the exact expression pattern and the biological relevance of the mammalian homologues of all three miRNAs remain unresolved (Roush, S. & Slack, F.J. (2008). The let-7 family of microRNAs. Trends Cell Biol. 18, 505-516.). However, mis-expression of *let-7* or *miR-125* have been observed in several human cancers, indicating an essential function of these miRNAs in controlling cell proliferation and differentiation (Roush, S. & Slack, F.J. (2008). The let-7 family of microRNAs. Trends Cell Biol. 18, 505-516.).

Recently, an intrinsic tumor eviction mechanism in *Drosophila* was described (Jiang, Y., Seimiya, M., Schlumpf, T.B., and Paro, R. (2018). An intrinsic tumour eviction mechanism in Drosophila mediated by steroid hormone signalling. Nature Communications 9, 1-9.). It was found that ecdysone signaling during metamorphosis, transforms tumorigenic *ph⁵⁰⁵* cells into non-tumorigenic "metamorphed" *ph⁵⁰⁵* cells, which become eliminated in adults. The let-7 miRNA and mir-125 were identified as important effectors of the process. However, it is still unknown which other transcripts can be derived from *let-7-C* in *Drosophila* and what their respective functions are.

### Objective problem to be solved

The objective problem to be solved is thus the provision of further RNA transcripts from *let-7-C* in *Drosophila* and the elucidation of the biological function and effect of these transcripts.

### Summary of the invention

The problem is solved by a RNA molecule having a sequence having at least 80% sequence identity to SEQ ID NO: 2.

Likewise, the problem is solved by a RNA molecule obtainable by transcription of a DNA sequence having at least 80% sequence identity to SEQ ID NO: 1 for use as medicament, preferably for use in the treatment of cancer.

Furthermore, the problem is solved by a pharmaceutical composition comprising as active ingredient a RNA molecule obtainable by transcription of a DNA sequence having at least 80% sequence identity to SEQ ID NO:1 or a vector loaded with a DNA sequence having at least 80% sequence identity to SEQ ID NO: 1.

### Brief description of the figures

**Fig. 1** shows that *let-7-C* may encode two non-coding transcripts. (A) Genomic region of the *let-7-C* in *Drosophila,* containing 3 exons (black boxes), one large intron and one small intron (dashed line). Transcriptome analysis of eye-antennal disc cells at 48 hours after pupal formation (metamorphed control) and metamorphed *ph⁵⁰⁵* cells by RNA-seq revealed many reads (black and grey bars indicate two replicates) mapping to the first intron. (B) *let-7-C* intron sequences can be observed in publicly available RNA-seq data in the Flybase. Notably, the intron sequences are enriched in samples from pupal stages, but not in embryonic, larval, and adult stages. (C) RNA-seq analysis of ecdysone-treated *ph⁵⁰⁵* culture cells showed many sequencing reads mapping in the first intron. Analysis of modENCODE data for RNA Pol II ChIP of adult heads and Kc167 cells reveals multiple regions with strong Pol II localization within the first intron, indicating potential transcription start sites. Based on the Pol II localization sites, as well as the sequencing reads, we named one IncRNA *let-A* and the other transcript *let-B.* In addition, the second half of the intron 1 was named I1B that was used in following experiments

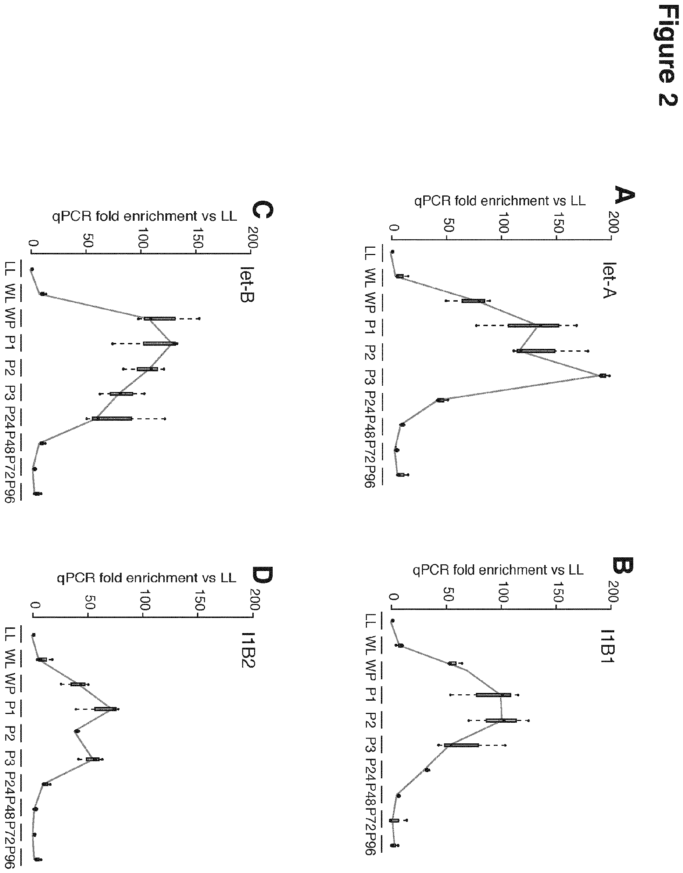

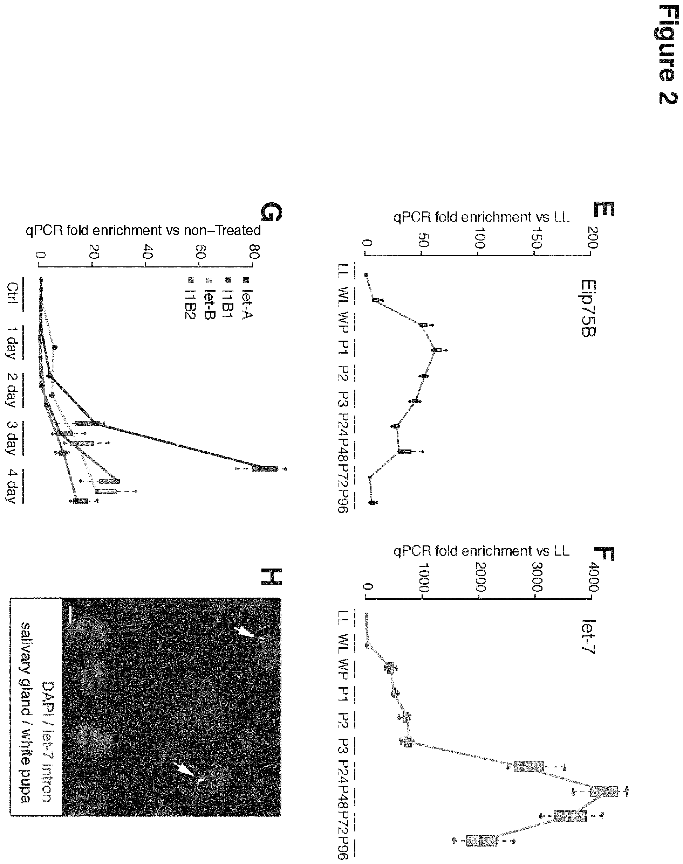

**Fig. 2** shows the dynamic expression of let-7-C ncRNAs during development. (A-E) qPCR quantification of the expression levels of different intronic regions of the let-7-C in whole larvae or pupae from different larval and pupal stages. Primers were used to amplify let-A coding region (A), intermediate region between let-A and let-B (B), let-B coding region (C), and 3' region outside of let-B (D). Eip75B, a known ecdysone-induce gene, was used here as a control (E). (F) qPCR quantification of the expression levels of different intronic regions of the let-7-C in ecdysone-treated *ph⁵⁰⁵* culture cells at different time points. Primers used were the same as in (A-D). (C) Expression dynamic of let-7 miRNA at the different developmental time points. (D) Confocal image of in situ hybridization of intron 1 sequences to whole salivary glands of white prepupae shows a strong signal (arrow) at the periphery of the nuclei (grey).

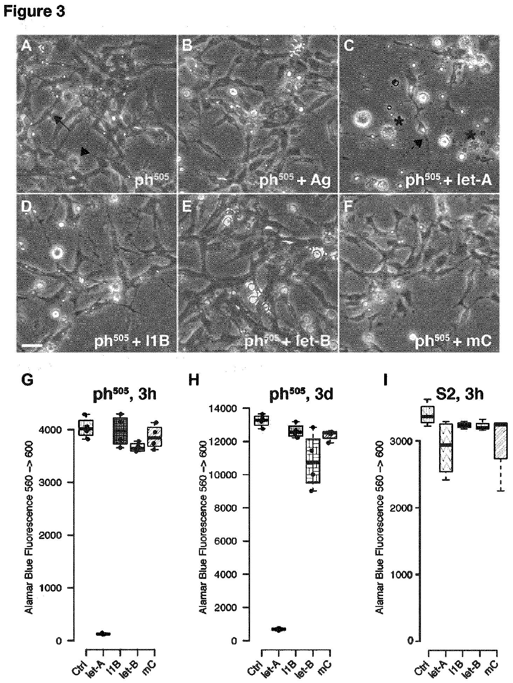

**Fig. 3** shows that induced expression of *let-A* resulted in rapid cell death in *ph⁵⁰⁵* cells. (A-F) Light microscope pictures showing *ph⁵⁰⁵* culture cells in different conditions: (A) untransfected; (B) silver-treated; (C) induced *let-A*; (D) induced I1B; (E) induced *let-B*; (F) induced mCherry (mC). All pictures showed the cells at two hours after induction. Arrow in (A) indicates a *ph⁵⁰⁵* cell which has an extended shape and attaches to the plate; arrowhead in (A) indicates a round shape floating *ph⁵⁰⁵* cell. Asterisks in (C) indicate dying cells; dashed arrow in (C) indicates a *ph⁵⁰⁵* cell with abnormal shape. Scale bar is 20 µm. (G) Alamar Blue quantification of cell viability at three hours (3h) after different RNAs were induced in *ph⁵⁰⁵* cells. This assay incorporates an oxidation-reduction indicator that both fluoresces and changes color in response to chemical reduction of medium resulting from cell growth. The fluorescence was measured and higher value correlated with more living cells in the culture. (H) Alamar Blue quantification of cell viability at three days (3d) after different RNAs were induced in *ph⁵⁰⁵* cells. (I) Alamar Blue quantification of cell viability at three hours after different RNAs were induced in S2 cells.

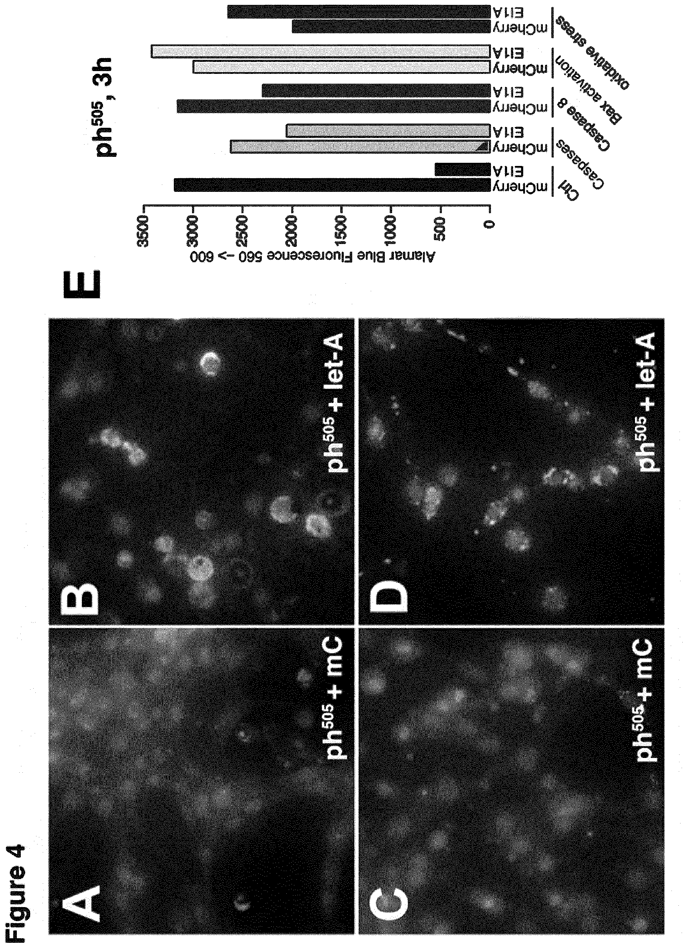

**Fig. 4** shows that *let-A* expression induced apoptotic cell death in *ph⁵⁰⁵* cells. (A) Annexin V staining in mCherry-expressing *ph⁵⁰⁵* cells. (B) Annexin V staining in *let*-*A*-expressing *ph⁵⁰⁵* cells showing many cells undergo apoptotic cell death. (C) CellRox staining in mCherry-expressing *ph⁵⁰⁵* cells. (D) CellRox staining in *let*-*A*-expressing *ph⁵⁰⁵* cells. (E) Cell viability measurements of *ph⁵⁰⁵* cells when different apoptosis inhibitors were applied during the induction of mCherry or *let-A.*

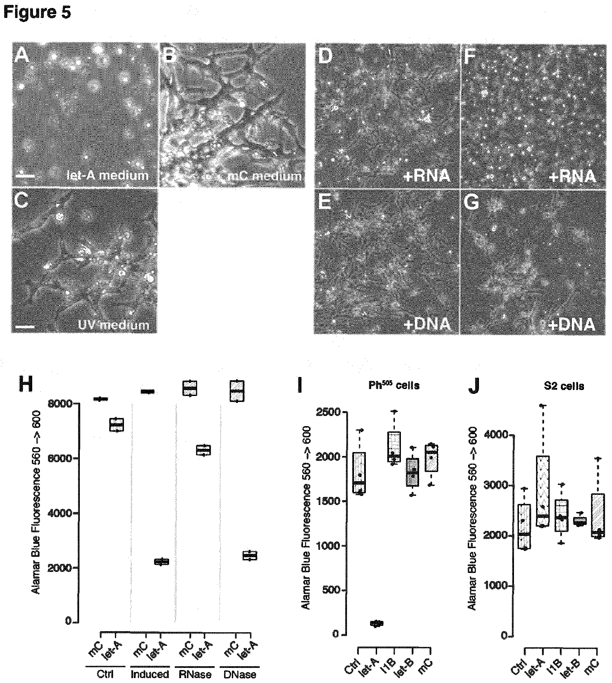

**Fig. 5** shows that cell death was induced by *let-A* RNA molecules. (A-C) Light microscope images showing *ph⁵⁰⁵* culture cells treated with let-A/medium (A), mCherry/medium (B), or UV medium (C). Scale bars are 20 µm. (D) Light microscope pictures showing *ph⁵⁰⁵* culture cells treated with purified RNA fraction from mCherry/medium. (E) Light microscope pictures showing *ph⁵⁰⁵* culture cells treated with purified DNA fraction from mCherry/medium. (F) Light microscope pictures showing *ph⁵⁰⁵* culture cells treated with purified RNA fraction from let-A/medium. (G) Light microscope pictures showing *ph⁵⁰⁵* culture cells treated with purified DNA fraction from let-A/medium. (H) Cell viability measurements of *ph⁵⁰⁵* cells when RNase or DNase were added to the medium during the induction of *let-A* or mCherry. Note that adding RNase could significantly increase cell viability in the culture, but DNase could not. (I) Cell viability measurements of *ph⁵⁰⁵* cells treated with RNAs purified from *let-A, let-B, I1B,* or mCherry/medium. Only purified RNA from let-A/medium was toxic to the cells. (J) Cell viability measurements of S2 cells treated with RNAs purified from *let-A, let-B, I1B,* or mCherry/medium. Purified RNA from let-A/medium was not toxic to S2 Cells.

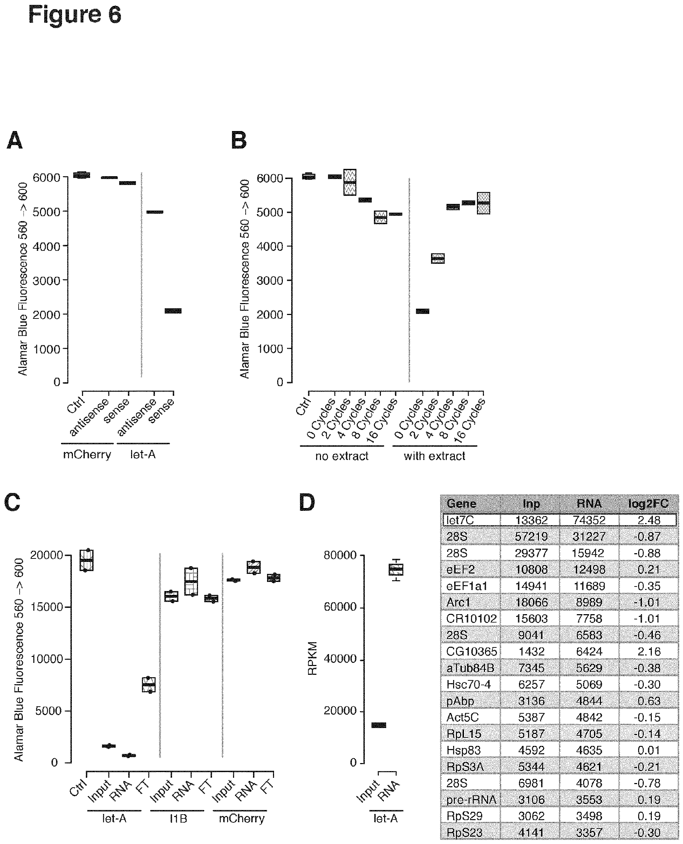

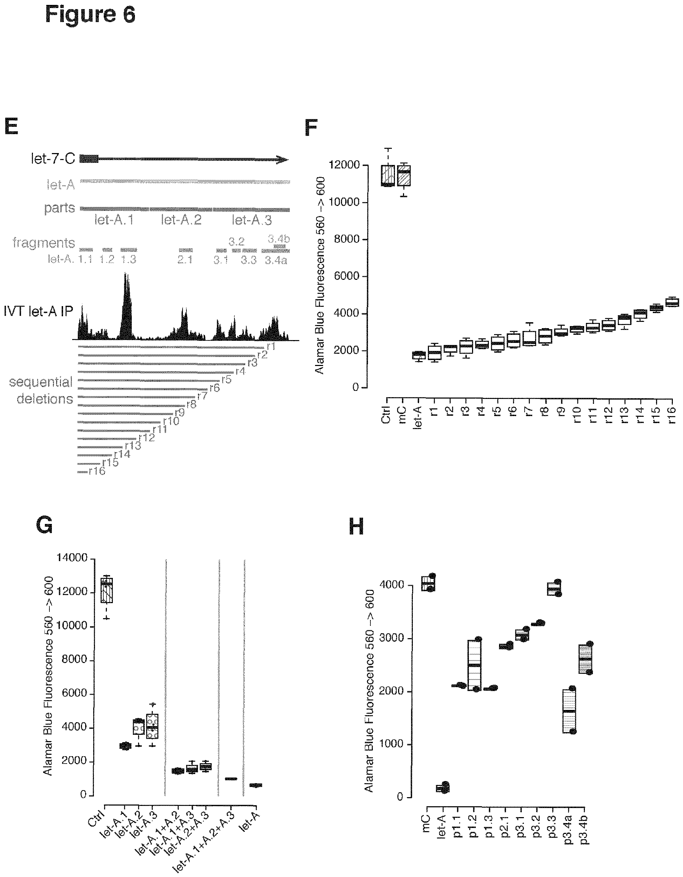

**Fig. 6** shows that cell toxicity was induced by *in vitro* transcribed full length *let-A* RNA. (A) Cell viability of *ph⁵⁰⁵* cells treated with in vitro transcribed sense or antisense *let-A* and mCherry RNAs. Only the sense *let-A* RNA was toxic to the cells. (B) Cell viability of *ph⁵⁰⁵* cells treated with sonicated *let-A* RNA. The RNA lost its toxicity with increasing number of sonication cycles. (C) Cell viability of *ph⁵⁰⁵* cells treated with in vitro transcribed *let-A, let-B*, or mCherry RNA. RNAs were transcribed with biotinylated ribonucleotides, incubated with cell extracts, and purified by affinity purification before applied to the cells. Input, without purification; RNA, affinity purified RNA; FT, flow through after affinity purification. (D) RNA-seq of the input or affinity purified in vitro transcribed RNA as in (C). Number of reads per kilobase million (RPKM) showed the enrichment of *let-A* after purification. Among the RNAs with the top number of sequencing counts, *let-7-C* had the most reads. (E) A map of the deletion constructs and shorter parts and fragments within *let-A* sequence. IVT let-A IP shows the sequencing reads enriched within *let-A.* (F) Cell viability of *ph⁵⁰⁵* cells transfected with constructs encoding a series of *let-A* deletion sequences. (G) Cell viability of *ph⁵⁰⁵* cells transfected with vectors encoding *let-A* parts p1-p3. (H) Cell viability of *ph⁵⁰⁵* cells transfected with vectors encoding *let-A* short fragments.

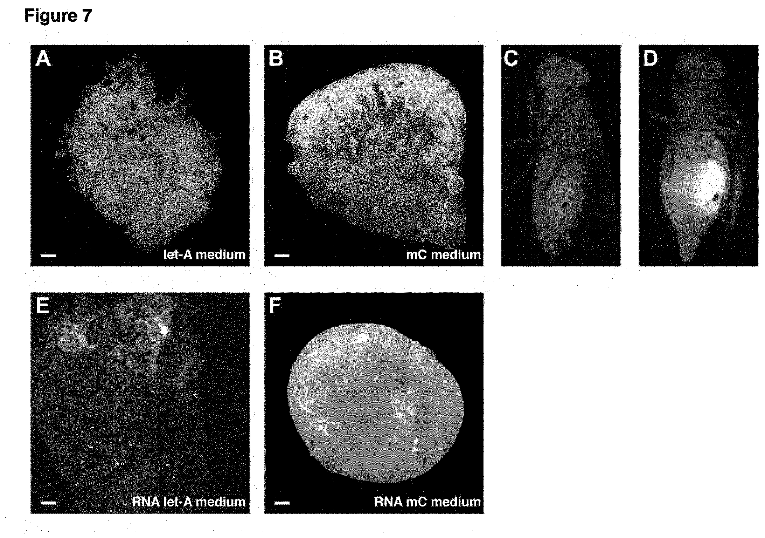

**Fig. 7** shows that *let-A* was toxic to in vivo growing *ph⁵⁰⁵* tumors. (A, B) Confocal images showing *ph⁵⁰⁵* tumor after 24 hours incubation in let-A/medium (A) or mCherry/medium (B). Note the tumor cells became dissociated and no longer formed a sphere structure after incubation in let-A/medium. Scale bars are 50 µm. (C, D) Adult host flies transplanted with let-A/medium-incubated *ph⁵⁰⁵* tumor (C) or mCherry/medium-incubated *ph⁵⁰⁵* tumor (D). let-A/medium-incubated *ph⁵⁰⁵* tumor could not grow, but mCherry medium-incubated *ph⁵⁰⁵* tumor could form tumors in the host flies. (E, F) Confocal picture showing *ph⁵⁰⁵* tumor after overnight incubation with purified RNAs from let-A/medium (E) or from mCherry/medium (F). Note the tumor cells became dissociated and no longer formed a sphere structure after incubation with purified RNA from let-A/medium. Scale bars are 50 µm.

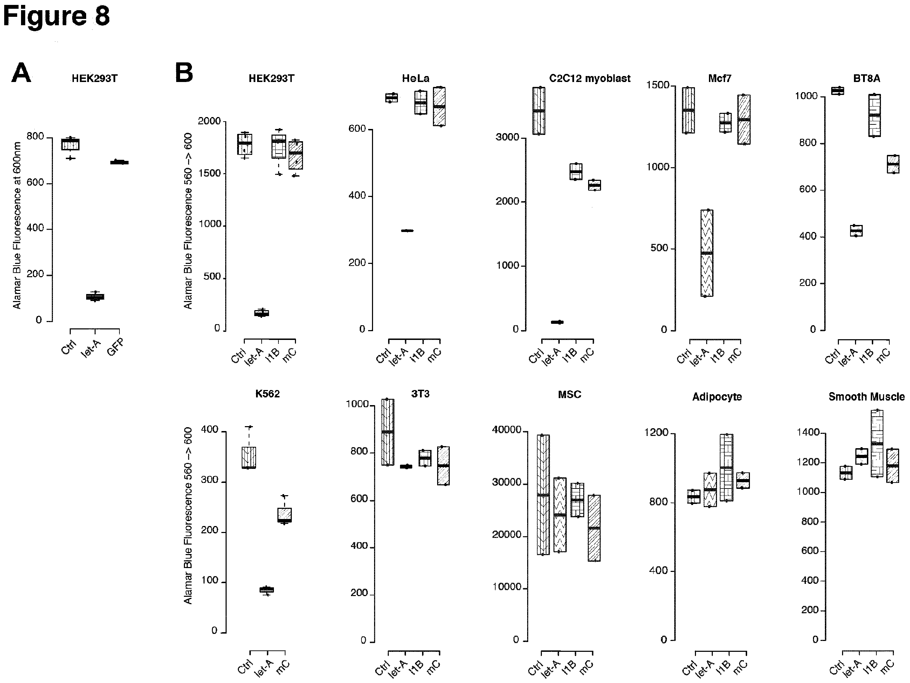

**Fig. 8** shows that *let-A* RNA is also toxic to mammalian cells. (A) Cell viability of HEK293T cells transfected with *Drosophila let-A* or GFP. Induced expression of *let-A* was toxic to HEK cells. (B) Cell viability of various mammalian cell lines treated with purified RNA from let-A/medium, I1B/medium, or mCherry/medium. The purified RNA from *let-A*/medium could kill HEK293T, HeLa, C2C12 myoblast, Mcf7, BT8A, and K562 cells.

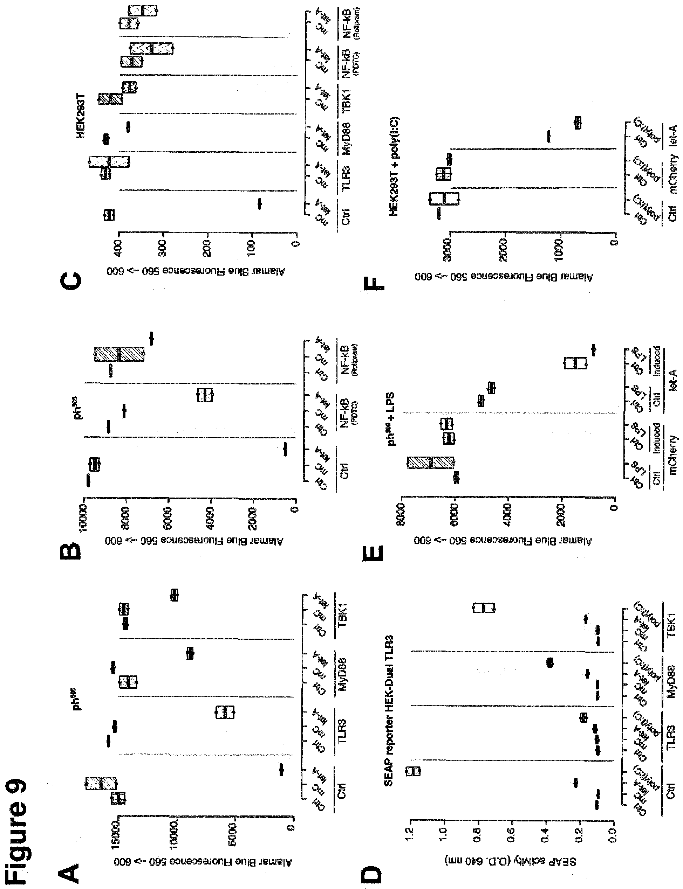

**Fig. 9** shows that Toll signaling pathway is required for *let-A* induced cell toxicity. (A) Cell viability of *ph⁵⁰⁵* cells treated with inhibitors against Toll signaling components TLR3, MyD88, and TBK1. Cells were first incubated with different inhibitors for one hour, then treated with purified RNAs from mCherry/medium or let-A/medium for three hours. (B) Cell viability of *ph⁵⁰⁵* cells treated with two NF-κB inhibitors. Cells were treated by the same processes as (A). (C) Cell viability of HEK393T cells treated with inhibitors against Toll signaling components. Cells were treated by the same inhibitors and processes as (A). (D) SEAP activity in HEK-Dual hTLR3 cells treated with purified RNA from let-A/medium or mCherry/medium, with or without Toll signaling inhibitors. (E) Cell viability of *ph⁵⁰⁵* cells with or without pre-treatment with LPS before *let-A* or mCherry expression was induced. Note that *let-A* induction could kill the LPS pre-treated cells even faster. (F) Cell viability of HEK393T cells with or without pre-treated with Poly(I:C), before treated with purified RNA from mCherry/medium or let-A/medium. let-A/medium purified RNA could kill the pre-treated cells much faster.

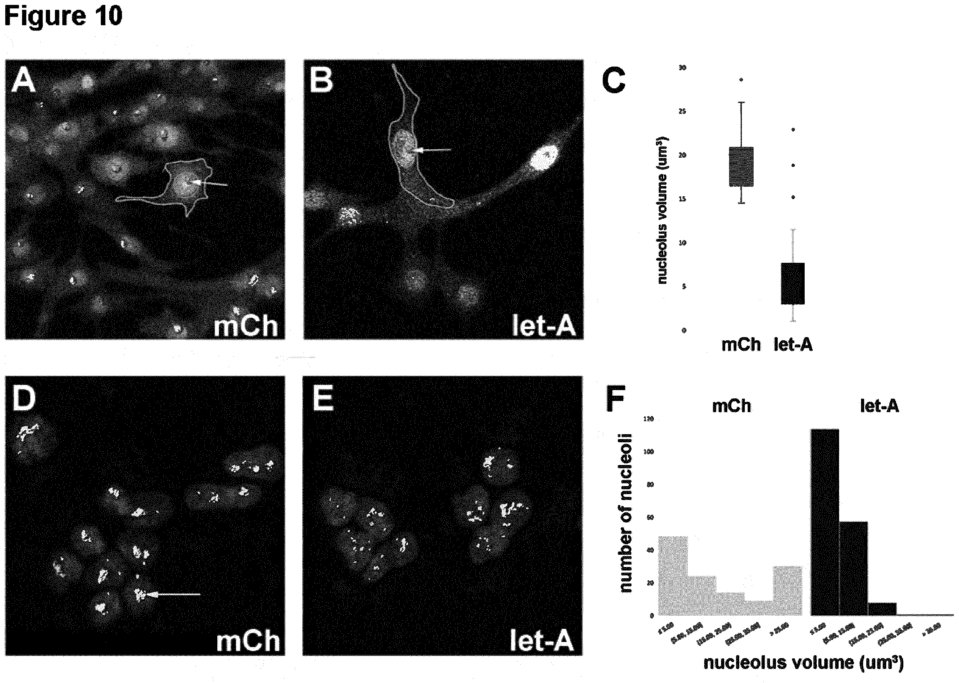

**Fig. 10** shows let-A/medium can reduce the nucleolus's volume in treated cells. (A, B) confocal images showing *ph⁵⁰⁵* cells treated with mCherry/medium (mCh) (A) or let-A/medium (let-A) (B). Immunostaining using Fibrillarin antibody (nucleolus). The boundary of a single cell and its nucleus are outlined, and the arrow points to the nucleolus stained by fibrillarin immunofluorescence. (C) Quantification of the nucleolus volume showing that the nucleolus volume was reduced in let-A/medium treated *ph⁵⁰⁵* cells. (D, E) confocal images showing HEK cells treated with mCh (D) or let-A (E). Immunostaining using Fibrillarin antibody (nucleolus). In mCh treated cells, each nucleus contains one large Fibrillarin staining (volume larger than 25 um³) and a few smaller ones. The arrow points to the nucleolus stained by fibrillarin immunofluorescence. But in let-A treated cells, the larger structure disappears and more smaller staining are observed in the nucleolus. (F) Quantification of the number of nucleoli with different volume in mCh or let-A treated HEK cells, showing that the nucleolus volume was reduced in let-A/medium treated cells.

### Detailed description of the invention

The invention relates to a RNA molecule having a sequence having at least 80% sequence identity to SEQ ID NO: 2.

The RNA molecule having a sequence according to SEQ ID NO: 2 is herein also referred to as *Drosophila let-A* IncRNA, *let-A* IncRNA or simply *let-A.* In a preferred embodiment, the RNA molecule is single-stranded.

The inventors found that induced expression of *let-A* results in rapid death of *Drosophila ph⁵⁰⁵* cancer cells and that dead cells further release this RNA, thus becoming toxic to neighboring cancer cells. In particular, it was found that when let-A RNA was over-expressed in cultured *ph⁵⁰⁵* cells, this results in rapid cell death of the entire population within a few hours. It was surprising that only a subset of the *ph⁵⁰⁵* cells in the culture were transfected by the virus vector and thus expressing the RNA, but still all the cells in the culture died. In addition, the culture medium also became toxic and could further induce cell death when applied to a new culture of *ph⁵⁰⁵* cells. These results indicate that cells expressing *let-A* release some molecules leading to the death of the untransfected cells in the same culture.

Without wanting to be bound by a mechanism, it is believed that *let-A* RNA may act through the Toll signaling pathway to induce apoptosis. Toll-like receptors (TLRs) are a family of membrane proteins that can induce an immune response upon recognition of microbial pathogens. First discovered in *Drosophila,* this family of proteins is evolutionarily conserved from flies to humans. In particular, several TLRs (TLR3, TLR7, TLR8, and TLR9) are known to recognize either double stranded or single stranded RNAs to initiate downstream signaling (Alexopoulou, L., Holt, A.C., Medzhitov, R., and Flavell, R.A. (2001). Recognition of double-stranded RNA and activation of NF-kappaB by Toll-like receptor 3. Nature. 413, 732-738.).

Transcriptome analyses of metamorphed cells derived from *ph⁵⁰⁵* tumor cells passing through metamorphosis showed, among other signaling pathways, a prominent activation of Toll signaling. The fact that this innate immune pathway is highly conserved also provides an explanation why mammalian cells also react to the exposure of the Drosophila *let-A* IncRNA.

The RNA molecule according to the invention has a sequence having at least 80% sequence identity to SEQ ID NO: 2. In the context of the invention, the term "sequences having at least 80% sequence identity" comprises sequences having at least 85 %, at least 90 %, at least 95 %, at least 96 %, at least 97 %, at least 98 %, at least 99 %, at least 99.5 %, most preferably 100 % sequence identity to the respective sequence. In all cases, the identity is the identity over the total length of the sequences.

In one aspect, the RNA molecule according to the invention is a variant of the RNA molecule having a sequence according to SEQ ID NO: 2, i.e. *let-A.* The term "variant" herein refers to biologically active derivatives of the respective RNA. In general, the term "variant" refers to molecules having a native sequence and structure with one or more additions, substitutions (generally conservative in nature) and/or deletions, relative to the native molecule, so long as the modifications do not destroy biological activity and which are "substantially homologous" to the reference molecule. In general, the sequences of such variants will have a high degree of sequence homology to the reference sequence, e.g., sequence homology of more than 50%, generally more than 60%-70%, even more particularly 80%-85% or more, such as at least 90%-95% or more, when the two sequences are aligned. In case of *let-A,* a variant thereof shows a similar capability to induce apoptosis in cancer cells, in particular *Drosophila ph⁵⁰⁵* cancer cells.

In a preferred embodiment, the RNA molecule has the same secondary structure as *let-A.* For example, the RNA molecule according to the invention has a double-stranded stem structure formed by complementary base pairing and a loop structure formed by unpairing bases. Within the stem structures, a small bulge loop or an internal loop may exist due to one or two unpairing bases. In addition, the RNA molecule may contain a pseudoknot structure.

The RNA molecules according to the invention are preferably long non-coding RNA (IncRNA) molecules. IncRNA molecules are herein defined as RNA molecules of more than 200 nucleotides without substantial ORFs.

In a further embodiment, the invention relates to a RNA molecule obtainable by transcription of a DNA sequence having at least 80% sequence identity to SEQ ID NO: 1 for use as a medicament. In a preferred embodiment, the RNA molecule is for the use in the treatment of cancer.

The term "obtainable by transcription of a DNA sequence" is herein understood as meaning that the RNA molecule is the transcription product or transcript of the respective DNA. Thus, the term "RNA molecule obtainable by transcription of a DNA sequence having at least 80% sequence identity to SEQ ID NO: 1" refers to any RNA molecule that can be transcribed from a DNA sequence having at least 80% sequence identity to SEQ ID NO: 1 or any processing product of such a RNA molecule. In particular, the RNA molecule according to the invention may have been processed by undergoing posttranscriptional modifications such as RNA methylation, folding, cleavage or wrapping into extracellular cargo vesicles such as exosomes.

The term "obtainable by transcription of a DNA sequence" is herein understood not to be limited with regard to the source of the RNA molecule. The RNA molecules according to the invention, including the RNA molecules used in the pharmaceutical compositions according to the invention, can be *in vivo* transcribed RNA molecules purified from cells or *in vitro* transcribed RNA.

The skilled person knows how RNA can be purified from cells or transcribed *in vitro.* For example, RNA purification can be performed by using TRIzol Reagent (Invitrogen, CA, catalogue no. 15596018). For this, samples are lysed and homogenised in the appropriate volume of TRIzol Reagent. Samples are incubated at room temperature for 5 minutes, then 1/5 of the volume of chloroform is added, incubated and mixed vigorously. To separate the phases samples are centrifuged for 15 minutes at 13 000 x g at 18°C. The aqueous phase containing the RNA is transferred to a new tube and RNA is precipitated using isopropanol. The resulting pellet is washed using ice cold 70% ethanol, air dried and dissolved in a suitable buffer.

The RNA molecules according to the invention may be purified from any eukaryotic cell expressing let-A artificially or naturally. In one embodiment, the RNA molecules according to the invention are purified from cells of *S*. *frugiperda, D. melanogaster, M. musculus* and *H. sapiens.* In a preferred embodiment, RNA molecules may be purified from Sf21, ph505, S2, CI.8, NIH-3T3, HEK293T or HeLa cells.

Likewise, RNA purification can be performed using commercially available kits such as RNeasy Mini Kit (Qiagen, Germany, catalogue no. 74106) or Dynabeads™ mRNA DIRECT™ Purification Kit (Invitrogen, CA, catalogue no. 61012).

For RNA *in vitro* transcription, numerous commercial kits are available, e.g. MEGAscript® T7 Transcription kit (Invitrogen, CA, catalogue no. AM1334). Likewise, RNA can be produced by oligonucleotide synthesis.

In a preferred embodiment, the RNA molecule obtainable by transcription of a DNA sequence having at least 80% sequence identity to SEQ ID NO: 1 is a RNA molecule having SEQ ID NO: 2.

The invention relates to use of these RNA molecules as a medicament, preferably for use in the treatment of cancer.

Surprisingly, the present inventors found the *Drosophila let-A* IncRNA to be toxic for different types of mammalian cancer cell lines, indicating an evolutionarily conserved oncolytic function. Consequently, the RNA molecules according to the invention are useful for the treatment of cancer.

The cancer may be selected from the group consisting of colon cancer, lung cancer, stomach cancer, esophageal cancer, pancreatic cancer, gallbladder cancer, renal cancer, bladder cancer, prostate cancer, testicular cancer, cervical cancer, endometrial cancer, choriocarcinoma, ovarian cancer, breast cancer, thyroid cancer, brain cancer, head and neck cancer, malignant melanoma, skin cancer, liver cancer, leukemia, lymphoma, multiple myeloma, chronic myelogenous leukemia, neuroblastoma and aplastic anemia.

The invention is also directed to methods of treatment of cancer, wherein the treatment comprises administration of a therapeutically effective amount of an RNA molecule according to the invention.

The term "administering" herein refers to administering an effective amount of the RNA molecules or the pharmaceutical compositions according to the invention, to a subject in need thereof.

Administering a nucleic acid, such as DNA or RNA molecule, in particular a IncRNA to a cell may be performed by transducing, transfecting, electroporating, translocating, fusing, phagocytosing, shooting or ballistic methods, etc., i.e., by any means by which a nucleic acid can be transported across a cell membrane.

In one aspect of the invention, the treatment induces differentiation and cell death in the cancer cells present in the cancer.

Differentiation of cancer cells may be measured by determining the nucleolus size by immunostaining with an antibody against the nucleolus protein Fibrillarin, followed by microscopy imaging and image processing. Cancer cells often have an enlarged nucleolus compared to normal cells. Another way to measure differentiation is to use cell markers specific for differentiated cells, for example, neuronal cell markers or muscle cell markers. The person skilled in the art is aware which markers can be used to this end. Differentiation of cancer cells can also be determined using transcriptomics, immunofluorescence or other cellular tests.

Surprisingly, treatment with let-A IncRNA resulted in a reduced volume of nucleolus in most of the cells. Thus, the RNA molecules of the invention are capable of conveying the nucleolar morphology of a healthy cell onto cancer cells.

The invention also relates to a pharmaceutical composition comprising as active ingredient a RNA molecule obtainable by transcription of a DNA sequence having at least 80% sequence identity to SEQ ID NO: 1.

It is herein understood that a pharmaceutical composition comprises a therapeutically effective amount of the active ingredient as well as pharmacological excipients. The person skilled in the art knows how to formulate a pharmaceutical composition comprising an RNA molecule. In particular, the person skilled in the art is aware of methods for stabilizing an RNA molecule in a pharmaceutical composition.

In the pharmaceutical composition according to the invention, the RNA molecule may be encapsuled in lipid nanoparticles, cellular or synthetic exosome mimics or in virus-like particles.

Herein, the term "lipid nanoparticles" is defined as molecules that are spherical in shape and comprise a solid lipid core stabilized by a surfactant. The core lipids can be steroids, fatty acids, acylglycerols, waxes, and combinations of them. Surfactants may be biological membrane lipids such as phospholipids, sphingomyelins and bile salts (e.g., sodium taurocholate). All of these may be utilized as stabilizers in the lipid nanoparticles used for pharmaceutical compositions of the invention.

Herein, the term "cellular or synthetic exosome mimics" is defined as nano-sized vesicles (exosomes), derived or purified from cells with modifications (cellular exosome mimics) or generated by artificial methods like cell extrusion (synthetic exosome mimics) that serve as cargos to deliver proteins, nucleic acids or other cellular components to neighboring or distant cells.

Herein, the term "virus-like particles" is defined as multiprotein structures that closely resemble the organization and conformation of viruses but contain no viral genetic material.

It may enhance the efficacy of the pharmaceutical composition or the RNA molecules according to the invention to activate the RNA molecule prior to administration. This can be achieved by incubating the RNA molecule with cell extracts. Therefore, in one aspect, the treatment comprises incubating the RNA molecule with cell extracts prior to administration. For example, the RNA molecules according to the invention may be incubated with cell extracts from cells from *S. frugiperda, D. melanogaster, M. musculus* and *H. sapiens.* In particular, the RNA molecules may be incubated with cell extracts fromSf21, ph505, S2, CI.8, NIH-3T3, HEK293T or HeLa cells.

The term "incubating with cell extracts" herein refers to a process in which cells are first homogenised in extraction buffer comprising protease and phosphatase inhibitors. In a second step, the thus obtained active cell lysate is mixed with the respective RNA molecules, e.g. the RNA molecules of the invention, for an appropriate amount of time, e.g. 5 to 15 min. Lastly, the RNA molecules are purified as described above.

Without wanting to be bound by a mechanism, it is believed that incubation with cell extracts activates and/or stabilizes the RNA molecules according to the invention and thus further enhances efficacy of the RNA molecules.

In another embodiment, the invention relates to a pharmaceutical composition comprising as active ingredient a vector loaded with a DNA sequence encoding a RNA molecule having at least 80% sequence identity to SEQ ID NO: 2. In one embodiment, the polynucleotide sequence has a sequence having at least 80% sequence identity to SEQ ID NO: 1.

The vector may be selected from the group consisting of a plasmid vector, a cosmid vector, or any other vector-based RNA expression system, a virus, and analogs thereof. The virus may be a retrovirus or a lentivirus. In a preferred embodiment, the virus is an adenovirus or an adeno-associated virus.

The term "loaded" is herein understood to mean that in case of the vector being a plasmid vector or a cosmid vector, said vector comprises said DNA sequence. In case the vector is a virus, the term is understood to mean that the virus carries said DNA sequence and is able to transduce cells with said DNA sequence.

The pharmaceutical compositions according to the invention are useful in the treatment of cancer.

The cancer may be selected from the group consisting of colon cancer, lung cancer, stomach cancer, esophageal cancer, pancreatic cancer, gallbladder cancer, renal cancer, bladder cancer, prostate cancer, testicular cancer, cervical cancer, endometrial cancer, choriocarcinoma, ovarian cancer, breast cancer, thyroid cancer, brain cancer, head and neck cancer, malignant melanoma, skin cancer, liver cancer, leukemia, lymphoma, multiple myeloma, chronic myelogenous leukemia, neuroblastoma and aplastic anemia.

### Examples

### Example 1

### Materials and methods

### Fly genetics

Fly strains were maintained on standard medium. All genetic experiments were performed at 25°C. To generate homozygous *ph⁵⁰⁵* MARCM clones, virgin females of *ph⁵⁰⁵ FRT19A*/*FM7 act-GFP* were crossed with *tub-Gal80 FRT19A; ey-flp act>STOP>Gal4 UAS-GFP* males.

To measure the expression of the IncRNA during development, we collected larvae and pupae (*w¹¹¹⁸*) at different developmental time points and extracted RNA for qPCR analyses. For larvae, third instar larvae staying in the food were collected as late larvae (LL); and wandering larvae were larvae leaving the food and wandering in the vial. For pupae, white pupae were collected and aged in a separate plate for 1, 2, 3, 24, 48, 72, and 96 hours.

The following fly strains were used in this study: (1) *Ore-R,* (2) *w¹¹¹⁸*, (3) *ph⁵⁰⁵ FRT19A*/*FM7 act-GFP,* (4) *tub-Gal80 FRT19A; ey-flp act>STOP>Gal4 UAS-GFP.*

### Transplantation

Transplantation experiments were carried out as previously described. In brief, 4-6 days old adult *w¹¹¹⁸* females were used as hosts (n>20 for each transplantation). The host flies were immobilized on an ice-cold metal plate and stuck on a piece of double-sided sticky tape, with their ventral sides up. The dissected tumor tissue was cut into small pieces of similar size and each piece was transplanted into the abdomen of one host using a custom-made glass needle. All transplantation was made under a GFP microscope to ensure labelled cells were injected into the hosts. After transplantation, host flies were allowed to recover at room temperature for 1-2 hours in fresh standard *Drosophila* medium before transferred to and maintained at 25°C.

To test the toxicity of the let-A medium on *in vivo* growing tumors, host flies carrying 1-week-old transplanted *ph⁵⁰⁵* tumors were dissected and the tumors were incubated with either control medium (mCherry medium) or let-A medium for 24 hours. The tumors were then cut into small pieces and re-transplanted into new host flies.

### ph⁵⁰⁵ cell line generation

In order to establish *ph⁵⁰⁵* cell line, two to three 1-week old transplanted *ph⁵⁰⁵* tumors were isolated from the host flies and dissected into small pieces in sterilized PBS following 3 times wash with Shields and Sang M3 Insect medium (US Biological) containing Penicillin/streptomycin(P/S). The cells were resuspended in 1ml of M3 (P/S) medium and plated into the 96 well plate 200µl per well. After the stable cell growth, single cell line was isolated using Terasaki plate (Greiner Bio-One). To confirm the cell line, genomic DNA was isolated and sequence analysis performed.

### RNA in situ in salivary glands

For single molecule FISH analysis (Fluorescence *in situ* Hybridization) a set of oligonucleotides spanning the first intron of *let-7-C* was designed using the Stellaris Probe Designer software (Biosearch Technologies). The oligonucleotides were ordered from Biosearch Technologies labeled with Quasar-670.

RNA FISH was preformed following the Stellaris protocol for suspension cells with minor modifications. Briefly, salivary glands from white pupa were dissected and washed in PBS and fixed with 4% formaldehyde in PBS for 10 min at room temperature. After washing with PBS samples were incubated with 70% EtOH for 24 hours at 4°C. Then, samples were washed in washing buffer (2xSSC, 10% formamide) at room temperature. For hybridization 125 nM of the probe set in hybridization buffer (Biosearch Technologies) was added and incubated overnight at 37°C. The next day, samples were washed twice for 30 min at 37°C with washing buffer, the second wash containing 500 ng/ml DAPI (Sigma-Aldrich). After one wash with PBS samples were mounted with Vectashield Mounting Medium (Vectorlabs).

### Cell culture and treatments

*Drosophila* Clone 8 (CI.8) and *ph⁵⁰⁵* cell lines were maintained at 25 °C in Shields and Sang M3 Insect medium (US Biological), supplemented with 2% Fetal Bovine Serum (FBS) (PAN Biotech), 2.5% fly extract, 5 µg/ml human insulin (Sigma), 100 U/ml penicillin and 100µg/ml streptomycin (Gibco, Life Technologies). S2 cells were cultured in Schneider's medium (Gibco, Life Technologies) supplemented with 10% FBS (PAN Biotech) at 25 °C. *Spodoptera frugiperda* Sf21 cells (Clontech) were propagated in Grace's medium (Gibco, Life Technologies) with 10% FBS (PAN Biotech) at 27°C. All mammalian cell lines except for the mesenchymal stem cells were grown in Dulbecco's Modified Eagle's Medium (DMEM) high glucose (Sigma) supplemented with 10% FBS (Sigma) and 2 mM L-glutamine (Gibco). For HEK-Dual hTLR3 cells (InvivoGen) the growth medium was additionally supplemented with 100 µg/ml Normocin, 100 µg/ml Hygromycin B and 50 µg/ml Zeocin (all from InvivoGen). Primary human bone marrow-derived mesenchymal stem cells were obtained from ATCC (PCS-500-012). They were maintained according to manufacturer's instructions and differentiated as previously described (Almalki, S.G. & Agrawal, D.K. (2016). Effects of matrix metalloproteinases on the fate of mesenchymal stem cells. Stem Cell Res Ther. 7(1): 129).

HEK293T cells were transfected with using Fugene HD (Promega) following manufacturer's instructions. After two days transcription was induced with 1 µg/ml tetracycline (Sigma) and cell viability was measured after overnight incubation.

SEAP activity was detected using QuantiBlue (InvivoGen) and the absorbance quantified on a Tecan Infinite M1000 PRO microplate reader at 650 nm.

For the ecdysone time course, cells were treated one day after seeding with 5 µM 20-Hydroxyecdysone (Sigma) and incubated for the specified time before RNA was extracted.

To induce apoptosis, *ph⁵⁰⁵* cells were treated twice with UV-C (90 mJ/cm2, 254 nm). For the UV treatment, cells were washed in PBS and irradiated with UV light in PBS, which was afterwards replaced by medium.

For the RNase and DNase treatment 100 µg/ml RNase mix (Roche) or 100 U/ml DNase I (Roche) and 100 µM AgNO3 were added together to the transduced cells. After 3 hours cell viability was measured using alamarBlue.

The following inhibitors were used: Apoptosis inhibitors: Pan-Caspase Inhibitor Z-VAD-FMK (Enzo Life Sciences) 50 µM; Caspase 8 Inhibitor Z-IETD-FMK (Enzo Life Sciences) 15 µM; Clusterin (secretory form, human recombinant, Enzo Life Sciences) 0.5 µM; oxidative stress was reduced with 1 mM Trolox (6-Hydroxy-2,5,7,8-tetramethylchromane-2-carboxylic acid, Sigma). Toll signaling pathway inhibitors: TLR3/dsRNA Complex Inhibitor (Sigma) 30 µM; MyD88 inhibitor T6167923 (Anawa) 50 µM; TBK1 inhibitor MRT67307 (Sigma) 2 µM; and the NF-kB inhibitors Rolipram (Abcam) 1 µM; pyrrolidine dithiocarbonate (PDTC, Sigma) 25 µM. For all the inhibitor experiments, cells were pretreated one hour with the respective inhibitor before induction or addition of the extracted medium. For induction, the cell viability was determined after three hours, for extracted medium treatment after one hour, for *Drosophila* cell lines and for mammalian cell lines after overnight incubation.

For activation of the Toll pathway cells were pretreated for one hour with 10 µg/ml LPS (Sigma) or 5 µg/ml poly(I:C) HMW (InvivoGen). Two hours after induction cell viability was measured with alamarBlue.

### Generation of recombinant baculoviruses

Cloning was performed following the strategy described in Lee et al. (2000). The *let-A* and *I1B* fragments were amplified from S2 cell genomic DNA using the Expand Long Template PCR System (Roche) and cloned in pBacPAK8_EGFP under a metallothionein promoter. *let-B* was cloned from pBacPAK8_EGFP_I1B. Viruses were generated, amplified, analyzed and harvested using the BacPAK Baculovirus Expression System (Clonetech) according to manufacturer's instruction.

### Cloning for mammalian cell expression

For inducible expression in mammalian cells, *let-A, let-B, I1B,* mCherry and EGFP were cloned from pBacPAK8 into pSBtet (Addgene) under the control of an inducible TRE promoter.

For in vitro transcription the fragments were cloned in pGEM-T Easy plasmids under the control of a T7 promoter.

### Transduction of Drosophila cells with baculoviruses

Cells were plated one day prior to transduction. For transductions cells were first washed twice with Grace's medium and incubated for two hours at room temperature with the virus inoculum. After removal of the virus, fresh medium was added and cells were incubated at 25°C for one more day before induction. Unless otherwise specified, transcription of the constructs was induced with 100 µM AgNO3 (Sigma). The conditioned medium was always harvested one day after induction.

### Cell viability measurement

Cell viability was measured after the indicated time using alamarBlue cell viability assay reagent (Thermo Fisher Scientific) following the manufacturer's instructions. Fluorescence at 650 nm was measured with Tecan Infinite M1000 PRO microplate reader.

### RNA extraction, cDNA synthesis and Quantitative real-time PCR

RNA from cells or medium was extracted using TRIzol (Invitrogen) following manufacturer's instructions. Reverse transcription was done with the first Strand synthesis kit (Fermentas) using oligo (dT)18 as a primer and qPCR was carried out with LightCycler 96 (Roche) using FastStart Essential DNA Green Master Mix (Roche). Expression levels were normalized to ATPasecf6.

For miRNA expression analysis, RNA was reverse transcribed using TaqMan miRNA Reverse Transcription kit (Applied Biosystems) and qPCR was performed using let-7 and 2S rRNA TaqMan MicroRNA Assay primer mix (Thermo Fisher Scientific) with the TaqMan Small RNA Assay (Applied Biosystems).

### DNA isolation

DNA was purified from conditioned medium by phenol-chloroform extraction.

*Annexin V and CellROX staining.*

To detect an early stage of apoptosis, cells were incubated one day after transduction with 2.5 mM CaCl2 (Sigma), 5 µl/ml Alexa Fluor-conjugated Annexin V (Biolegend) and 1 µg/ml Hoechst33342 for 10 minutes at room temperature and then induced with 100 µM AgNO3 and imaged on several time points. To detect the presence of ROS, CellROX DeepRed Reagent (Life Technologies) was used. Before induction, cells were incubated for 30 minutes with 2.5 µM CellROX reagent. Nuclei were also stained with 1 µg/ml Hoechst33342. Images were taken using a Leica MZ16 FA fluorescent microscope.

### in vitro RNA transcription and purification

RNA was transcribed from linearized pGEM plasmids using the MEGAscript T7 Transcription kit (Ambion) following the manufacturer's instructions. The IVT products were purified using the MEGAclear kit (Ambion). Concentration and size of the transcripts were determined using TapeStation 4200 (Agilent).

Active cellular extract was prepared as described (Crevel, G. & Cotterill, S. (1991). DNA replication in cell-free extracts from Drosophila melanogaster. EMBO J. 10, 4361-4369.) with slight modifications. Cells were washed twice with PBS and then 43*10^6 cells were dounced on ice in a dounce homogenizer (Pestle B) in extraction buffer (10 mM HEPES, pH 7.5, 10 % v/v ethyleneglycol, 250 mM sucrose, 100 mM NaCl, 2.5 mM MgCI2, 1 mM EDTA, 2 mM DTT, 2 mM ATP, PhosSTOP Phosphatase Inhibitor Cocktail (Roche) and cOmplete™ Protease Inhibitor Cocktail (Roche)). The lysate was centrifuged for 10 min at 20'000g at 4°C. The supernatant was taken and total protein concentration was determined by Pierce BCA Protein Assay Kit (Thermo Fisher Scientific). 35 µl of extract (about 4 mg/ml protein) was mixed with 375 ng of IVT RNA (diluted in 15 µl extraction buffer), incubated at 25 °C for 15 minutes and then the RNA was purified by TRIzol extraction.

Biotinylated RNA was produced by *in vitro* transcription in the presence of Desthiobiotin-16-UTP (TriLink Biotechnologies). The resulting transcripts were incubated with active cellular extract. To isolate the biotin labelled RNA, the mixture was incubated with 20 µl of Dynabeads MyOne Streptavidin T1 (Invitrogen) for 15 minutes at 25 °C. Afterwards, another 20 µl beads were added and again incubated 15 minutes at 25 °C. Next, the beads were washed twice with extraction buffer and eluted in elution buffer (extraction buffer with 2 mM avidin (IBA)) at 25°C for 15 minutes. The eluates and flow-through were purified by TRIzol extraction.

The purified RNA was sequenced by first depleting ribosomal RNA with the Ribo-Zero Gold rRNA Removal Kit and then subjecting the remaining RNA to the Illumina TruSeq Stranded Library Prep Kit. The resulting libraries were sequenced on a NextSeq500 at paired end 38.

### Immunohistochemistry and antibodies

Tumours were fixed in 2% paraformaldehyde (in 1xPBS) for 25 minutes at room temperature, and washed several times in PBST (1xPBS with 0.5% Triton X-100). Tumours were incubated overnight with primary antibodies at 4°C, followed by several washes at room temperature, incubated with secondary antibodies at 4°C overnight. After several washes, samples were incubated with DAPI (1:200 in PBST) at room temperature for 20 minutes, then mounted in Vectashield and stored at -20°C before imaging.

Primary antibody used in this study was: chicken anti-green fluorescent protein (GFP) (1:1000; Abcam ab13970); Secondary antibodies were: Alexa 488-conjugated anti-chicken (1:500; Molecular Probes).

### Microscopy

Immunofluorescent images were recorded on a Leica TCS SP8 confocal microscope. Adult flies with transplanted tumours were taken on a Nikon SMZ1270 microscope. Images were processed using ImageJ, Imaris, Photoshop, and Adobe Illustrator.

### Transcriptome analyses

Transcriptome analyses were performed by mRNA sequencing. RNAs were isolated from the *ph⁵⁰⁵* culture cells at 16 hours after treated with ecdysone (5 µM 20-Hydroxyecdysone (Sigma)). RNA was extracted using an Arcturus PicoPure RNA Isolation kit (Applied Biosystems), library prepared with SMARTSeq2 NexteraXT and sequenced on an Illumina NextSeq2500 or NextSeq500.

### Bioinformatics analyses

Short reads were aligned to BDGP dm6 genome assembly using TopHat 2.0.12 for Bowtie 2.2.3 with parameters "--very-sensitive" and a segment length of 18 for the IVT reads. From the aligned reads, differential expression was called using R 3.5.1 and edgeR 3.24.3.

RPKM values for external miRNA expression data were retrieved from FlyBase (Release 2018_06).

### Example 2

### The let-7 complex encodes two putative non-coding RNAs

In our previous study, we discovered an endogenous tumor eviction mechanism in *Drosophila* (Jiang et al., 2018). Ecdysone signaling during metamorphosis, transforms tumorigenic *ph⁵⁰⁵* cells into non-tumorigenic "metamorphed" *ph⁵⁰⁵* cells, which become eliminated in adults. The *let*-7 miRNA and *mir-125* were identified as important effectors of the process. The *let-7* complex (*let-7-C*) in *Drosophila* is located on the second chromosome, the primary transcript encoding besides *let-7* and *mir-125* also *mir-100* spans ∼17 kb of genomic region and consisting of three exons and two introns (Fig. 1A). During a detailed transcriptome analysis of the metamorphed *ph⁵⁰⁵* cells by RNA-seq, to our surprise, we observed many reads mapping to the intronic region of *let-7-C* (Fig. 1A). These reads were particularly enriched in the 5' region of the first intron in one group of samples, and in the second half of the first intron in another (Fig. 1A). This indicates that the *let-7-C* may encode, besides the pri-RNA for the miRNAs, additional non-coding transcripts. To determine if these new non-annotated transcripts are present in other transcriptome database, we searched publicly available RNA-seq data in Flybase, which had been generated from various tissues and cells at different developmental stages. Indeed, the intronic sequences can also be observed in a few samples and cell lines (Fig. 1B). Interestingly, the intronic sequences were detected particularly in samples from pupal stages, but not in embryonic, larval, or adult stages, suggesting that the expression of these transcripts might be related to the expression of ecdysone signaling at metamorphosis (Fig. 1B).

In our previous study, we showed that larval *ph⁵⁰⁵* mutant cells can continue to proliferate and give rise to neoplastic tumors after being transplanted into adult host flies (Jiang et al., 2018). We isolated the transplanted tumors from adult hosts, dissociated the tumors, cultured the dissociated cells in fly medium, and thereby established a *ph⁵⁰⁵* primary cell line. To test if ecdysone can induce the expression of the non-coding transcripts in *let-7-C,* we treated *ph⁵⁰⁵* cell culture with ecdysone overnight, isolated the polyadenylated RNAs, and performed transcriptome analysis by RNA-seq. Similarly, this analysis showed many reads mapping to the second half of the first intron (Fig. 1C), suggesting ecdysone signaling can induce the expression of certain non-coding transcripts. Furthermore, an analysis of modENCODE data for RNA Pol II ChIP of adult heads and Kc167 cells reveals multiple regions with strong Pol II localization within the first intron (Fig. 1C). These observations suggest the *let-7-C* locus is more complex and encodes at least two IncRNA transcripts in addition to the primary transcript for the three miRNAs (Fig. 1C). Based on the Pol II localization sites, as well as the sequencing reads, we named one IncRNA *let-A* which consists of the first exon and the first half of the intron, and the other transcript *let-B* which consists of sequences in the second half of the first intron (Fig. 1C). Interestingly, three previously discovered ecdysone responsive elements (EcRE), which are required for the ecdysone-induced expression of *let-7-C,* are all located in the *let-B* transcript (Fig. 1C).

### In vivo expression of the two IncRNAs during development

To further examine if the two IncRNAs in the *let-7-C* are expressed *in vivo,* we treated *ph⁵⁰⁵* culture cells with ecdysone, collected RNAs at several time points, and quantified the expression of several regions within the first intron using different primer pairs by qPCR. Compared to the untreated *ph⁵⁰⁵* cells, the expression of both transcripts could be detected after exposure to ecdysone (Fig. 2A). The expression of *let-B* transcript was the first to be observed one day after ecdysone treatment, while *let-A* remained at basal levels (Fig. 2A). The expression level of *let-B* was similar for the first two days, and slightly increased in day three and four (Fig. 2A). On the other hand, the expression level of *let-A* was lower than *let-B* during the first two days after ecdysone treatment, but continuously increased thereafter (Fig. 2A). This shows that the IncRNA *let-B,* containing all three EcREs, is the first to be expressed in response to ecdysone and *let-A* is expressed at a later time point.

Next, we analyzed how the two IncRNAs are expressed during fly development. As the expression of the *let-7-C* locus is regulated by ecdysone signaling at the larva to pupa transition, we collected wild-type larvae and pupae at different developmental time points during the larva to pupa transition and after pupal formation, extracted RNA, and performed qPCR for different regions in the *let-7* intron (Fig. 2B). At late 3^{rd} larval instar (LL) and wandering larval (WL) stage, both of the two IncRNAs were expressed at a basal level (Fig. 2B). In the white pupae (WP), expression of both *let-A* and *let-B* started to increase (Fig. 2B). Similar to the ecdysone-treated culture cells, the fold-change of *let-B* expression was higher than that of *let-A* in the beginning, suggesting *let-B* was the first to be expressed (Fig. 2B). During the first 3 hours after pupae formation (APF) (P1-P3), the expression of *let-B* did not increase. In contrast, *let-A* expression continued to increase during the early hours of pupariation (Fig. 2B). The expression of both IncRNAs decreased at 24 hours APF, and we could only detect basal levels at 48 hours, 72 hours, and 96 hours APF (Fig. 2B). The expression dynamics of the two IncRNA looks very similar to that of the known ecdysone responsive gene *Eip75B* (Fig. 2B), but shows significant differences to the expression pattern of the miRNA *let-7* (Fig. 2C). *In situ* hybridization of both *let-A* and *let-B* sequences to whole salivary glands of white prepupae show a strong signal at the periphery of the nuclei, reflecting most probably the highly active site of synthesis (Fig. 2D). These results show that the two IncRNAs are expressed transiently during early pupal stage in normal development and exhibit a different expression profile compared to that of the *let-7* miRNA.

### Example 3

### Effects of induced expression of the two IncRNAs in culture cells

To characterize the potential function of the two IncRNAs, we transduced *ph⁵⁰⁵* culture cells with a silver-inducible viral vector that expresses either *let-A* or *let-B* IncRNA. In addition, we transduced either mCherry as control, or the entire second half of the first intron (named *I1B*) (Fig. 1C). In untransfected cells (Fig. 3A) or *ph⁵⁰⁵* cell cultures treated only with silver (Fig. 3B), most cells had either an extended shape and attached to the plate (arrow in 3A), or a round shape and floated in the medium (arrowhead in 3A), with only few dead cells. However, when we induced the expression of *let-A,* surprisingly almost all the *ph⁵⁰⁵* cells died within three hours (Fig. 3C, asterisks), with few remaining cells having an abnormal shape (Fig. 3C, dashed arrow). When inducing *let-B, I1B,* or mCherry in *ph⁵⁰⁵* cells, no such effect was observed (Fig. 3D-F). We used the alamarBlue assay to quantify cell viability when different RNAs were activated in *ph⁵⁰⁵* cells. In contrast to the untreated control cells, the cell viability was significantly reduced at three hours after *let-A* was expressed in *ph⁵⁰⁵* cells (Fig. 3G). However, the cell viability was not affected when *let-B*, *I1B*, or mCherry was induced (Fig. 3G). To test if any of these RNAs might have a long-term effect, we measured the cell viability at three days after induction. As expected, most of the cells were dead when *let-A* was induced (Fig. 3H). While the expression of *let-B* only weakly reduced the cell viability, the induction of *I1B* or mCherry did not affect the cell viability at all (Fig. 3H). These results show that the expression of IncRNA *let-A* is toxic to the *ph⁵⁰⁵* culture cells, but *let-B* is not. As *let-A* showed such a strong phenotype to cultured cells, we decided to focus on this IncRNA for further investigation.

Interestingly, the effect of *let-A* seemed to be cell type specific. When we transduced the RNAs in S2 or CI8 cells and induced the expression, the cells did not die and the viability was not affected (Fig. 3I).

To determine how cells died upon *let-A* expression, we performed immunostaining for the apoptosis marker Annexin V. In the control culture with mCherry expressed only very few dead cells could be observed (Fig. 4A). In contrast, in *let-A* expressing cultures, as early as 40 minutes after induction, most of the cells were undergoing apoptotic cell death (Fig. 4B). Next, we tested if the cells die due to oxidative stress using the CellRox assay. Expression of mCherry did not induce any change in oxidative stress in the *ph⁵⁰⁵* cells (Fig. 4C). However, when *let-A* was expressed in the *ph⁵⁰⁵* cells, a significant increase in cellular oxidative stress could be observed (Fig. 4D). We next applied several apoptosis inhibitors against pan-caspases, caspase 8, Bax, or oxidative stress inhibitor to the *ph⁵⁰⁵* cells during *let-A* expression, and in all conditions, inhibition of apoptotic cell death resulted in more surviving cells in the culture (Fig. 4E). All together, these results show that expression of *let-A* in *ph⁵⁰⁵* cells can induce a rapid apoptotic cell death.

### Cellular toxicity is induced by the let-A RNA molecule

As only a small subset of *ph⁵⁰⁵* cells was transduced by the viral vector, it was surprising to observe that almost all the cells in the culture died within a short time after *let-A* induction (Fig. 3G). We speculated that the transduced cells released some molecules, therefore making the medium toxic, further inducing the death in the untransfected cells in the culture. To test this, we induced the expression of *let-A* (or mCherry RNA as control) in *ph⁵⁰⁵* cells, collected the medium (henceforth called let-A/medium or mCherry/medium), and added the collected medium to another plate of untreated *ph⁵⁰⁵* cells. Adding the let-A/medium led to the death of most *ph⁵⁰⁵* cells within the hour (Fig. 5A). Conversely, adding mCherry/medium had no effect (Fig. 5B). To test whether the medium toxicity was due to cell apoptosis in general, we killed untreated *ph⁵⁰⁵* cells by UV, collected the medium, and added it to a new plate of *ph⁵⁰⁵* cells. Unlike the let-A/medium, the UV/medium had no effect on the *ph⁵⁰⁵* cells (Fig. 5C), indicating the toxicity of the medium is due to the induced expression of *let-A.*

To characterize the effector molecule in the medium, we induced the expression of *let-A* in *ph⁵⁰⁵* cells and collected the medium after most cells had died. We next isolated either RNA or DNA from the medium, purified twice by Trizol extraction, and added the purified fractions to *ph⁵⁰⁵* cells. The purified RNA or DNA from the mCherry/medium did not show any effect to *ph⁵⁰⁵* culture cells (Fig. 5D, E). However, the purified RNA from let-A/medium was able to kill all the cells (Fig. 5F), whereas DNA was not (Fig. 5G), indicating that the toxicity was mediated by RNA molecules. To further confirm this result, we added RNase or DNase in the medium during the induction. Clearly, adding RNase to the medium increased the cell viability but DNase did not, confirming the effector to be an RNase-sensitive molecule (Fig. 5H). Furthermore, cell viability tests showed that the toxicity was due to the specific expression of *let-A,* because purified RNA from the medium of other RNAs (*let-B, I1B,* or mCherry) would not kill *ph⁵⁰⁵* cells (Fig. 5I). The toxicity of the RNA was also cell-specific similarly to the *let-A* transductions as there was no effect when S2 cells were treated with the purified RNA from *let-A* medium (Fig. 5J).

### Cell toxicity can be induced by in vitro transcribed full length let-A RNA

To further test if the toxicity was caused by *let-A* itself, we *in vitro* transcribed (ivt) sense and antisense *let-A* or mCherry, and tested their toxicity on *ph⁵⁰⁵* cells. When *ph⁵⁰⁵* cells were treated with isolated ivt *let-A* RNA, the viability was not affected. However, when the ivt *let-A* RNA was first incubated with cell extracts, then added to *ph⁵⁰⁵* cells, the cells could be killed (Fig. 6A). In addition, only RNA with the sense sequence could kill the cells, but RNA with the antisense sequence could not (Fig. 6A). Moreover, when ivt *let-A* RNA was sonicated before incubation and application to *ph⁵⁰⁵* cells, it was no longer able to kill *ph⁵⁰⁵* cells (Fig. 6B). These results indicate the effector molecule is *let-A,* however, requiring further processing and/or modifications to induce the cell toxicity.

To further confirm this, we *in vitro* transcribed biotinylated *let-A,* incubated with cell extracts, and isolated the RNA by biotin-streptavidin affinity purification. The purified *let-A* RNA showed stronger toxic effect on *ph⁵⁰⁵* cells compared to the input RNA (Fig. 6C). In contrast, neither *I1B* nor mCherry RNA that were *in vitro* transcribed and processed by the same procedure was able to kill the *ph⁵⁰⁵* culture cells (Fig. 6C). We next did total RNA-seq with the affinity purified RNA. As shown in Fig. 6D, after affinity purification *let-A* sequence was enriched compare to the input sample. Among the RNAs with the highest number of sequencing counts, *let-7-C* had the most sequencing reads (Fig. 6D). Together, these results show that *let-A* RNA is indeed the molecule that induces apoptosis in *ph⁵⁰⁵* cells. To exert this toxicity, it needs to be modified and/or folded to a particular secondary structure.

### Example 4

### ph⁵⁰⁵ tumor tissue can be extinguished by let-A/medium and purified let-A RNA

Since *ph⁵⁰⁵* culture cells can be killed by the *let*-*A*/medium and by purified RNA, we hypothesized that the RNA may also be toxic to *in vivo* growing tumors. We first transplanted imaginal discs containing *ph⁵⁰⁵* mutant clones into adult host flies and collected the tumors growing in those flies. We dissected host flies carrying 1-week-old tumors and incubated the tumors with either control medium (mCherry medium) or *let-*A/medium for 24 hours. The tumor tissue became dissociated after incubation with *let-*A/medium (Fig. 7A). In contrast, the cells in the tumor incubated in the control medium still form tumor spheres and the bulk of tumor tissue does not show visible changes (Fig. 7B). In addition, after re-transplantation into adult host flies, *let-A*/medium-incubated tumors were no longer able to grow in host flies (Fig. 7C), but the control medium-incubated tumors still gave rise to the formation of tumors (Fig. 7D). These results show that the let-A/medium is also toxic to *in vivo* formed tumors.

Next, we tested if RNA purified from the *let*-*A*/medium has the same effect. Similarly, tumors incubated with the *let-A*/medium-purified RNA became dissociated (Fig. 7E) and could not form tumors after re-transplantation. In contrast, tumors incubated overnight with RNA from control medium remained tumorigenic and were able to grow after re-transplantation (Fig. 7F).

### Example 5

### let-A RNA exerts its toxic effect also on mammalian cells

As the IncRNA *let-A* can induce rapid apoptosis in Drosophila cancer cells, it is intriguing to test if this RNA has the same effect on mammalian cells. Therefore, we transfected HEK293T cells with Drosophila *let-A* and measured the cellular viability. To our surprise, Drosophila *let-A* was also toxic to the HEK293T cells and most cells died after overnight incubation (Fig. 8A). Furthermore, the RNA fraction purified from *let*-*A*/medium was also able to kill the HEK293T cells, but RNAs from the *I1B* or *mCherry*/medium could not (Fig. 8B). We next treated several other mammalian cell lines with RNAs purified from the *let-*A/medium, including HeLa, C2C12 myoblast, Mcf7, BT8A, and K562 cells. Indeed, purified RNA was able to kill all those cell lines (Fig. 8B) with RNAs purified from *I1B*/medium or *mCherry*/medium not having the same effect (Fig. 8B). These results show that Drosophila *let-A* has an evolutionarily conserved oncolytic function active also in mammalian cells.

### Example 6

### The Toll signaling pathway is activated during the cellular response to let-A

To uncover the underlying mechanism in both *Drosophila* and mammalian cells, we investigated what signaling pathways might be involved during this process. To test if the Toll signaling pathway is required for the *let-A* induced cell death, we applied several inhibitors that targeted different components in *ph⁵⁰⁵* cells (Fig. 10A). When the receptor TLR3 was blocked, an increase in cell viability was observed (Fig. 9A). When inhibitors against more downstream components were applied, including the adaptor protein MyD88 and the kinase TBK1, we observed a further increase in cell viability (Fig. 9A). In addition, we used two inhibitors for the most downstream transcription factor NF-κB, which also resulted in a better cell survival (Fig. 9B). Next, we used the same inhibitors to block Toll signaling in mammalian HEK293T cells. Similarly, the treatment could inhibit *let-A* induced toxicity in the HEK cells and significantly increase the cell viability (Fig. 9C).

We next used a HEK-Dual hTLR3 cell line that expresses SEAP (Secreted embryonic alkaline phosphatase) as a reporter for Toll signaling. The SEAP activity was low in the untreated condition, but increased upon treatment with Poly(I:C) (polyinosine-polycytidylic acid), a known inducer of the Toll signaling pathway (Fig. 9D). Treating HEK-Dual hTLR3 cells with Poly(I:C) could increase the SEAP activity in these cells (Fig. 9D). When HEK-Dual hTLR3 cells were treated with mCherry/medium, we did not observe an increase in SEAP activity (Fig. 9D). However, treating cells with let-A/medium could increase SEAP activity in the cells (Fig. 9D), indicating that Toll signaling was induced. Furthermore, when the Toll pathway inhibitors were added, SEAP activity was strongly reduced in both Poly(I:C) treated and *let-A* treated cells (Fig. 9D).

Lastly, we pre-treated *ph⁵⁰⁵* cells with LPS (lipopolysaccharide), which is an inducer of Toll signaling but does not cause cell death (Fig. 9E), to sensitize the cells to other activators of Toll signaling. When mCherry was induced in LPS pre-treated *ph⁵⁰⁵* cells, it did not have any effect (Fig. 9E). In contrast, when *let-A* was induced in LPS pre-treated *ph⁵⁰⁵* cells, it killed the cells even faster and all cells died within one hour (Fig. 9E). Similarly, Poly(I:C) pre-treated HEK cells also became more sensitive to *let-A* and showed a further reduced viability compared to cells untreated with Poly(I:C) (Fig. 9F). Together these results show that Toll signaling pathway is required for *let-A* induced cell death in both *Drosophila* and human cancer cells.

## Claims

1. A RNA molecule having a sequence having at least 80% sequence identity to SEQ ID NO: 2.

2. A RNA molecule obtainable by transcription of a DNA sequence having at least 80% sequence identity to SEQ ID NO: 1 for use as a medicament.

3. A RNA molecule obtainable by transcription of a DNA sequence having at least 80% sequence identity to SEQ ID NO: 1 for use in the treatment of cancer.

4. The RNA molecule for use according to claim 2, wherein the cancer is selected from the group consisting of colon cancer, lung cancer, stomach cancer, esophageal cancer, pancreatic cancer, gallbladder cancer, renal cancer, bladder cancer, prostate cancer, testicular cancer, cervical cancer, endometrial cancer, choriocarcinoma, ovarian cancer, breast cancer, thyroid cancer, brain cancer, head and neck cancer, malignant melanoma, skin cancer, liver cancer, leukemia, lymphoma, multiple myeloma, chronic myelogenous leukemia, neuroblastoma and aplastic anemia.

5. The RNA molecule for use according to any of claims 2 to 4, wherein the cancer comprises cancer cells and wherein the treatment induces differentiation and/or cell death in the cancer cells.

6. The RNA molecule for use according to any of claims 2 to 5, wherein the treatment comprises incubating the RNA molecule with cell extracts prior to administration, in particular with cell extracts from cells from *S. frugiperda, D. melanogaster, M. musculus* or *H. sapiens.*

7. The RNA molecule for use according to any of claims 2 to 5, wherein the RNA molecule has a sequence having at least 80% sequence identity to SEQ ID NO: 2.

8. A pharmaceutical composition comprising as active ingredient a RNA molecule obtainable by transcription of a DNA sequence having at least 80% sequence identity to SEQ ID NO: 1.

9. The pharmaceutical composition according to claim 7, wherein the RNA molecule is RNA purified from cells or *in vitro* transcribed RNA.

10. The pharmaceutical composition according to claim 7, wherein the RNA molecule is encapsuled in lipid nanoparticles, cellular or synthetic exosome mimics or in virus-like particles.

11. The pharmaceutical composition according to any of claims 8 to 10 wherein the RNA molecule has a sequence having at least 80% sequence identity to SEQ ID NO: 2.

12. A pharmaceutical composition comprising as active ingredient a vector loaded with a DNA sequence having at least 80% sequence identity to SEQ ID NO: 1.

13. The pharmaceutical composition according to claim 12, wherein the vector is selected from the group consisting of a plasmid vector, a cosmid vector, a virus, and analogs thereof.

14. The pharmaceutical composition according to claim 13, wherein the RNA molecule is activated by incubating the RNA molecule with cell extracts prior to administration, in particular with cell extracts from cells from *S. frugiperda, D. melanogaster, M. musculus* or *H. sapiens.*

15. The pharmaceutical composition according to any of claims 7 to 14 for use in the treatment of cancer.
